(19) Europäisches Patentamt
European Patent Office
Office européen des brevets

(11) **EP 3 840 706 B1**

(12) **EUROPÄISCHE PATENTSCHRIFT**

(45) Veröffentlichungstag und Bekanntmachung des Hinweises auf die Patenterteilung:
**26.03.2025 Patentblatt 2025/13**

(21) Anmeldenummer: **19756349.7**

(22) Anmeldetag: **19.08.2019**

(51) Internationale Patentklassifikation (IPC):
**A61F 13/15** *(2006.01)* **A61F 13/49** *(2006.01)*

(52) Gemeinsame Patentklassifikation (CPC):
**A61F 13/15756; A61F 13/49015**

(86) Internationale Anmeldenummer:
**PCT/EP2019/072088**

(87) Internationale Veröffentlichungsnummer:
**WO 2020/038860 (27.02.2020 Gazette 2020/09)**

(54) **VERFAHREN ZUR HERSTELLUNG VON INKONTINENZWEGWERFWINDELN**

METHOD FOR PRODUCING DISPOSABLE INCONTINENCE DIAPERS

PROCÉDÉ POUR LA FABRICATION DE COUCHES JETABLES POUR INCONTINENCE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.08.2018 DE 102018120495**

(43) Veröffentlichungstag der Anmeldung:
**30.06.2021 Patentblatt 2021/26**

(73) Patentinhaber: **Paul Hartmann AG**
**89522 Heidenheim (DE)**

(72) Erfinder:
• **REISEL, Robert**
**89518 Heidenheim (DE)**
• **HIPP, Thomas**
**89522 Heidenheim (DE)**
• **NUVOLONI, Veronica**
**89081 Ulm (DE)**
• **KNECHT, Theresia**
**73433 Aalen (DE)**
• **DWORSKY, Tobias**
**89428 Syrgenstein (DE)**

(74) Vertreter: **Paul Hartmann AG**
**Patents & Licensing**
**Paul-Hartmann-Straße 12**
**89522 Heidenheim (DE)**

(56) Entgegenhaltungen:
EP-A1- 1 719 484 WO-A1-2007/042084
WO-A1-2011/101773 DE-A1- 102015 226 814

**Beschreibung**

Die vorliegende Erfindung betrifft ein Verfahren zum Herstellen einer Vielzahl von Inkontinenzwegwerfwindeln des offenen Typs für Erwachsene.

Derartige Inkontinenzwegwerfwindeln sind bekannt und weisen häufig einen Hauptteil, bestehend aus einem Vorderbereich, einem Rückenbereich und einem in Windellängsrichtung dazwischen liegenden zwischen den Beinen eines Benutzers zu liegen kommenden Schrittbereich auf, wobei der Hauptteil meist bereits einen Absorptionskern umfasst, und mit an hinteren seitlichen Längsrändern des Rückenbereichs beidseitig angefügten, voneinander separaten Windelseitenteilen, welche sich in Windelquerrichtung über die seitlichen Längsränder des Hauptteils hinaus erstrecken und den Vorderbereich und den Rückenbereich im angelegten Zustand des Artikels miteinander verbinden.

Die Windelseitenteile werden vorzugsweise im Cut&Place-Verfahren (Slip-Cut) direkt, beidseitig an den Hauptteil, also das Chassis des Hygieneartikels angefügt. Diese Fertigungstechnologie erlaubt es, die Windelseitenteile aus einem anderen Material zu fertigen als den Hauptteil des Hygieneartikels. Beispielsweise können die Windelseitenteile zumindest bereichsweise elastisches Material umfassen, wohingegen der Hauptteil aus im Wesentlichen undehnbarem Material gefertigt sein kann.

Die aus Fertigungssicht effizienteste und einfachste sowie kostengünstigste Art zur Bereitstellung von Windelseitenteilen ist die Abtrennung der Windelseitenteile von einer endlosen Flachmaterialbahn. Die Flachmaterialbahn kann dabei aus einem Verbundmaterial bestehen, derart, dass endlose und sich je nach Bedarf in ihren Materialeigenschaften wie beispielsweise Dehnbarkeit oder Atmungsaktivität unterscheidende Materialbahnen an ihren jeweiligen Längsrändern aneinandergefügt sind.

WO2008/036706A1 offenbart ein Verfahren zur Herstellung von Windelseitenteilen mittels einer in Querrichtung einnutzigen Windelseitenteilbahn, welche in der Querrichtung drei voneinander differenzierbare Bereiche aufweist. Die Windelseitenteile werden durch Trennen der Windelseitenteilbahn in Querrichtung vereinzelt, insbesondere derart, dass aufeinanderfolgend gebildete Windelseitenteile in der Ebene um 180° gedreht ausgerichtet sind.

WO2011/101773A1 offenbart ein Verfahren zur Herstellung von Hygieneartikeln, wobei eine in Querrichtung zweinutzige Windelseitenteilbahn einer Hauptteilbahn zugeführt wird, wobei die Windelseitenteilbahn derart hergestellt wird, dass äußere Seitenteilabschnitte von einer ersten vorgefalteten Materialbahn abgetrennt und getaktet an eine zweite vorgefaltete Materialbahn angefügt werden. Das getaktete Anfügen der äußeren Seitenteilabschnitte an die zweite Materialbahn bringt eine aufwendige Prozessführung mit sich.

WO96/03952A1 offenbart ein Verfahren zur Herstellung einer Windel, wobei Windelohrabschnitte bestehend aus einem ersten und einem zweiten Ohr sowie einem dazwischen eingefügten Brückenmaterial von einer endlosen zusammengesetzten Materialbahn abgetrennt und derart an den Windelhauptteil angefügt werden, dass das Brückenmaterial den Windelhauptteil überfängt. Das Brückenmaterial wirkt sich nachteilig auf die Herstellkosten der Windel aus.

WO96/31180A1 offenbart ein Verfahren zur Herstellung eines Verschlusssystems für Hygieneartikel, wobei eine endlose Verschlussmaterialbahn an zwei endlose Flachmaterialbahnen angefügt wird, derart, dass die Verschlussmaterialbahn den zentralen Bereich einer zweinutzigen Windelseitenteilbahn bildet. Vor der Vereinzelung muss die zweinutzige Windelseitenteilbahn durch Schneiden, insbesondere mäanderförmiges Schneiden durch Verschlussmaterial hindurch in zwei einnutzige Windelseitenteilbahnen getrennt werden.

WO2007/042084A1 offenbart ein Verfahren zur Herstellung einer Windel mit angefügten Windelseitenteilen umfassend elastische Bereiche. Dazu wird ein elastisches Material auf ein Trägermaterial aufgebracht und das Trägermaterial bereichsweise überdehnt.

Der vorliegenden Erfindung liegt die Aufgabe zu Grunde, ein verbessertes Verfahren zum Herstellen von derartigen oder ähnlichen Inkontinenzwegwerfwindeln bereitzustellen, das kostengünstig und prozesstechnisch vorteilhaft realisierbar ist.

Diese Aufgabe wird gelöst durch ein Verfahren zur Herstellung von Inkontinenzwegwerfwindeln, wobei eine jeweilige Inkontinenzwegwerfwindel eine Windellängsrichtung und eine Windelquerrichtung aufweist, und wobei die Inkontinenzwegwerfwindel einen mit einem Saugkörper versehenen Hauptteil aufweist, wobei der Hauptteil einen Rückenbereich mit einem ersten hinteren seitlichen Längsrand und mit einem zweiten hinteren seitlichen Längsrand aufweist, und wobei an den ersten hinteren seitlichen Längsrand ein sich in der Windelquerrichtung über den ersten hinteren seitlichen Längsrand des Hauptteils hinaus erstreckendes erstes elastisches Windelseitenteil angefügt ist, und wobei an den zweiten hinteren seitlichen Längsrand ein sich in der Windelquerrichtung über den zweiten hinteren seitlichen Längsrand des Hauptteils hinaus erstreckendes zweites elastisches Windelseitenteil angefügt ist, umfassend die folgenden Schritte

- Fördern einer ersten Materialbahn, einer zweiten Materialbahn, einer dritten Materialbahn, einer vierten Materialbahn und einer fünften Materialbahn, wobei die erste Materialbahn eine Breite B1 und einen ersten Materialbahnrandbereich und einen zweiten Materialbahnrandbereich aufweist und ein in einer Querrichtung der ersten Materialbahn im Wesentlichen undehnbares Anfügematerial umfasst oder daraus besteht, wobei die zweite Materialbahn eine Breite B2 und einen dritten Materialbahnrandbereich und einen vierten Materialbahnrandbereich aufweist und ein erstes in einer Querrichtung der zweiten Materialbahn zumindest bereichsweise elastisches Material umfasst oder daraus besteht, wobei die dritte Materialbahn eine Breite B3 und einen fünften Materialbahnrandbereich und einen sechsten Materialbahnrandbereich aufweist und ein zweites in einer Querrichtung der dritten Materialbahn zumindest bereichsweise elastisches Material umfasst oder daraus besteht, wobei die vierte Materialbahn eine Breite B4 und einen siebten Materialbahnrandbereich und einen achten Materialbahnrandbereich aufweist und ein erstes in einer Querrichtung der vierten Materialbahn im Wesentlichen undehnbares Verschlussträgermaterial umfasst oder daraus besteht, und wobei die fünfte Materialbahn eine Breite B5 und einen neunten Materialbahnrandbereich und einen zehnten Materialbahnrandbereich aufweist und ein zweites in einer Querrichtung der fünften Materialbahn im Wesentlichen undehnbares Verschlussträgermaterial umfasst oder daraus besteht, und wobei die Materialbahnrandbereiche der ersten, zweiten, dritten, vierten und fünften Materialbahn derart aneinandergefügt werden, dass die erste, zweite, dritte, vierte und fünfte Materialbahn eine in ihrer jeweiligen Querrichtung zweinutzige Windelseitenteilbahn bilden, derart, dass ein zentraler Bereich der zweinutzigen Windelseitenteilbahn durch die erste Materialbahn gebildet wird, dass ein erster äußerer Bereich der zweinutzigen Windelseitenteilbahn durch die vierte Materialbahn gebildet wird, dass ein zweiter äußerer Bereich der zweinutzigen Windelseitenteilbahn durch die fünfte Materialbahn gebildet wird, und dass ein in der Querrichtung der zweinutzigen Windelseitenteilbahn zwischen dem ersten äußeren Bereich und dem zentralen Bereich angeordneter erster elastischer Bereich durch die zweite Materialbahn gebildet wird, und dass ein in der Querrichtung der zweinutzigen Windelseitenteilbahn zwischen dem zweiten äußeren Bereich und dem zentralen Bereich angeordneter zweiter elastischer Bereich durch die dritte Materialbahn gebildet wird,

- Trennen der zweinutzigen Windelseitenteilbahn in einer Längsrichtung der zweinutzigen Windelseitenteilbahn und durch den zentralen Bereich der zweinutzigen Windelseitenteilbahn hindurch, zur Bildung einer ersten und zweiten jeweils in ihrer jeweiligen Querrichtung einnutzigen Windelseitenteilbahn,
- Vereinzeln von Windelseitenteilabschnitten durch Trennen der ersten und der zweiten einnutzigen Windelseitenteilbahn in der jeweiligen Querrichtung der ersten und zweiten einnutzigen Windelseitenteilbahn,
- Zuführen einer Windelhauptteilbahn mit einem ersten Windelhauptteilbahnrand und einem zweiten Windelhauptteilbahnrand,
- Anfügen der Windelseitenteilabschnitte an ersten Bereichen des ersten Windelhauptteilbahnrands und an zweiten Bereichen des zweiten Windelhauptteilbahnrands,
- Vereinzeln der Inkontinenzwegwerfwindeln durch Trennen der Windelhauptteilbahn in einer Querrichtung der Windelhauptteilbahn.

Durch das erfindungsgemäße Verfahren wird die kostengünstige Bereitstellung einer in der Querrichtung zweinutzigen und in Querrichtung elastisches Material aufweisenden Windelseitenteilbahn ermöglicht, welche durch verfahrenstechnisch vorteilhaft zu führende Trenn- und Vereinzelungsschritte prozesssicher in Windelseitenteilabschnitte getrennt werden kann.

Soweit nicht anders beschrieben bezieht sich im Rahmen der vorliegenden Erfindung die Ein-, Zwei- oder Dreinutzigkeit einer jeweiligen Bahn auf deren Querrichtung, also auf eine Richtung orthogonal zur jeweiligen Längsrichtung der Bahn.

Der Faktor des "Nutzens" (ein-, zwei-, drei-) bezieht sich auf die Anzahl der Segmente - wie beispielsweise im Falle der Windelseitenteilbahn auf die Anzahl der Windelseitenteilabschnitte - welche von der Bahn in der jeweiligen Richtung gebildet oder abgetrennt werden können.

Dass die erste Materialbahn und/oder das von der ersten Materialbahn umfasste Anfügematerial - vermittels welchem die Windelseitenteilabschnitte an den Windelhauptteilbahnrand angefügt werden - im Wesentlichen undehnbar ausgebildet ist, bietet zudem den Vorteil, dass die Stabilität der Fügeverbindung zwischen der Windelhauptteilbahn und dem Anfügematerial der Windelseitenteilabschnitte erhöht ist. Dass die vierte und/oder fünfte Materialbahn und/oder das von der vierten und/oder fünften Materialbahn bevorzugt umfasste Verschlussträgermaterial, an das nach einer bevorzugten Variante Verschlusselemente angefügt werden, im Wesentlichen undehnbar ausgebildet ist, erleichtert außerdem die Herstellung einer Fügeverbindung zwischen Verschlusselementen und einem jeweiligen Windelseitenteilabschnitt.

Das Verschlussträgermaterial der vierten und/oder der fünften Materialbahn und/oder das Anfügematerial der ersten Materialbahn umfasst oder besteht vorzugsweise aus einem Vliesstoffmaterial, wie Spinnvlies, Meltblownvlies, Kardenvlies, Spunlacevlies oder Through Air bonded Kardenvlies oder Kombinationen hiervon. Besonders bevorzugt sind Spinnvliesmaterialien (Spunbondvliesstoffe) und/oder Meltblownvliesmaterialien, insbesondere Laminate aus Spunbond- (S) und Meltblown- (M) Vliesstoffen oder Vliesschichten wie SMS-, oder SSMS- oder SMMS-, oder SSMMS- oder SSMMSS-Laminate.

Es hat sich weiter als vorteilhaft erwiesen, wenn das Vliesstoffmaterial zumindest eine Rezepturkomponente auf Basis eines thermoplastischen Polymers enthält, wie Polyethylen PE, Polypropylen PP oder Polyethylenterephthalat PET oder Mischungen davon. Vliesstoffmaterialien umfassend Rayon, Cellulose, Polyamid PA und Mischungen davon sind ebenfalls denkbar.

Das Vliesstoffmaterial hat vorteilhaft ein Flächengewicht von 10 - 70 g/m$^2$, weiter vorzugsweise von 20 - 60 g/m$^2$, weiter vorzugsweise von 30 - 50 g/m$^2$.

Nach einer bevorzugten Ausführungsform umfassen oder bestehen das erste und das zweite Verschlussträgermaterial aus demselben Material, insbesondere aus demselben Vliesstoffmaterial. Nach einer weiteren bevorzugten Ausführungsform umfassen oder bestehen das Anfügematerial und das erste und/oder das zweite Verschlussträgermaterial aus demselben Material, insbesondere aus demselben Vliesstoffmaterial. Das hat den Vorteil, dass Anfügematerial und Verschlussträgermaterial, insbesondere zwei, weiter insbesondere drei der Materialbahnen ausgewählt aus der Gruppe welche umfasst die erste Materialbahn, die vierte Materialbahn und die fünfte Materialbahn, aus einer einzigen, insbesondere in der Querrichtung zwei- oder dreinutzigen Materialbahn, insbesondere durch Trennen dieser Materialbahn in der Längsrichtung, insbesondere wie weiter unten näher beschrieben ist, bereitgestellt werden können.

Bevorzugt umfassen oder bestehen das erste und das zweite elastische Material aus demselben Material.

Das erste und/oder das zweite elastische Material der zweiten und/oder der dritten Materialbahn umfasst oder besteht weiter vorzugsweise aus einem Flachmaterial insbesondere aus einem Vliesstoff, einer Folie, einem textilen Material, einem Schaumstoff oder Kombinationen davon. Insbesondere umfasst oder besteht das erste und/oder das zweite elastische Material aus einem Laminat aus zwei oder mehreren der genannten Flachmaterialien. Weiter vorzugsweise kann das erste und/oder das zweite elastische Material aus einem Verbund von elastischen Komponenten und nicht elastischen Komponenten gebildet sein, insbesondere aus einem Verbund aus einer elastischen Folie oder einem elastischen Vliesstoff oder einem elastischen Schaumstoff oder elastischen Fäden wie Lycra- oder Spandex- oder Elasthan-Fäden mit einem nicht elastischen und/oder dehnbaren Vliesstoff oder Schaumstoff.

Im Falle eines Verbundes von elastischen Komponenten und nicht elastischen Komponenten kann der Verbund dadurch als elastisches Material bereitgestellt werden, dass die nicht elastische Komponente dehnbar ausgebildet ist und solchen Falls der elastischen Dehnung der elastischen Komponente keinen oder nur wenig Widerstand entgegensetzt. Eine weitere Möglichkeit ist, die Komponenten im sogenannten, dem Fachmann an sich bekannten stretch-bonding Verfahren zu verbinden, mithin die elastische Komponente in vorgedehntem Zustand mit der unelastischen Komponente zu verbinden. Eine weitere Möglichkeit, eine Elastifizierung eines Verbundes von elastischen Komponenten und nicht elastischen Komponenten zu erreichen, besteht darin, dass der Verbund - vorzugsweise durch eine als "ring rolling" bekannt gewordene Technologie - "aktiviert" wird. Diese Technologie ist beispielsweise in EP 0 650 714 A1 beschrieben. Durch "ring rolling" wird ein an sich nicht dehnbares Verbundmaterial, beispielsweise ein Vlies-/Folien-Laminat durch übermäßige Auslenkung zwischen gegeneinander kämmenden Walzen überdehnt. In diesem überdehnten Zustand bringt das zuvor an sich nicht dehnbare Material des Laminates einer Längung im Wesentlichen keinen Widerstand entgegen. Durch Kombination mit einem elastisch dehnbaren Element innerhalb eines derartigen Laminates kann somit eine elastische Dehnbarkeit in dem entsprechend behandelten Bereich erreicht werden.

Nach einer bevorzugten Variante des Verfahrens weist die in der Querrichtung zweinutzige Windelseitenteilbahn eine in der Längsrichtung der zweinutzigen Windelseitenteilbahn verlaufende erste Symmetrieachse auf. Weiter insbesondere erfolgt die Trennung der zweinutzigen Windelseitenteilbahn entlang der ersten Symmetrieachse.

In einer bevorzugten Variante wird der dritte Materialbahnrandbereich der zweiten Materialbahn an den ersten Materialbahnrandbereich der ersten Materialbahn angefügt, und der fünfte Materialbahnrandbereich der dritten Materialbahn an den zweiten Materialbahnrandbereich der ersten Materialbahn angefügt, derart dass die erste, zweite und dritte Materialbahn gemeinsam eine Kompositbahn bilden, wobei sich die Materialien der ersten, zweiten und dritten Materialbahn in einer Längsrichtung der Kompositbahn endlos erstrecken, und wobei die Kompositbahn einen ersten Kompositbahnrandbereich und einen zweiten Kompositbahnrandbereich aufweist, und wobei der siebte Materialbahnrandbereich der vierten Materialbahn an den ersten Kompositbahnrandbereich der Kompositbahn angefügt wird, und wobei der neunte Materialbahnrandbereich der fünften Materialbahn an den zweiten Kompositbahnrandbereich der Kompositbahn angefügt wird, derart dass die Kompositbahn gemeinsam mit der vierten und fünften Materialbahn die in ihrer Querrichtung zweinutzige Windelseitenteilbahn bilden, wobei sich die Materialien der vierten und fünften Materialbahn in einer Längsrichtung der Windelseitenteilbahn endlos erstrecken.

Nach einer bevorzugten Ausführungsform des Verfahrens erweist es sich als vorteilhaft, das Anfügen der zweiten und dritten Materialbahn an die erste Materialbahn gleichzeitig, also insbesondere im selben Verfahrensschritt vorzunehmen.

Nach einer weiteren bevorzugten Variante erweist es sich als vorteilhaft, das Anfügen der vierten und fünften Materialbahn an die Kompositbahn gleichzeitig, also insbesondere im selben Verfahrensschritt vorzunehmen.

Nach einer bevorzugten Variante des Verfahrens weist die Kompositbahn eine in der Längsrichtung der Kompositbahn verlaufende zweite Symmetrieachse auf.

Nach einer bevorzugten Ausführungsform weisen das erste und das zweite elastische Windelseitenteil jeweils mindestens ein Verschlusselement auf.

Nach einer weiteren bevorzugten Variante werden die jeweiligen Verschlusselemente insbesondere getaktet und mittels dem Fachmann an sich bekannter Applikationstechniken wie beispielsweise im Cut&Place-Verfahren (Slip-Cut) insbesondere an erste Bereiche eines ersten Windelseitenteilbahnrandes und an zweite Bereiche eines zweiten Windelseitenteilbahnrandes angefügt.

Dabei erweist es sich als vorteilhaft, wenn jedes Windelseitenteil genau ein Verschlusselement aufweist. Es handelt sich bei den Verschlusselementen typischerweise um eine Lasche aus einem ein- oder mehrschichtigen Flachmaterial, welches ausgehend von einer in der Regel um einen distalen Längsrand des betrachteten Windelseitenteils nach innen auf das Windelseitenteil eingefalteten Konfiguration in eine nach außen ausgefaltete Betriebsstellung entfaltbar ist. Alternativ ist denkbar, dass das Verschlussträgermaterial eines jeweiligen Windelseitenteils innerhalb des distalen Bereichs des Windelseitenteils ein funktionales Verschlusselement ("Patch") aufweist. In an sich bekannter und daher nicht näher zu beschreibender Weise ist ein jeweiliges Verschlusselement mit klebenden und/oder mechanisch haftenden Bereichen, Schichten oder Elementen ausgestattet, wie zum Beispiel Haken/Schlaufen-Materialien. Sofern das Windelseitenteil genau ein Verschlusselement aufweist, so erweist es sich als vorteilhaft, wenn dieses Verschlusselement in der Längsrichtung des Windelseitenteils ungefähr mittig und in einem distalen Bereich des Windelseitenteils vorgesehen ist. Weiter erweist es sich als vorteilhaft, wenn das betreffende Verschlusselement eine Erstreckung in der Längsrichtung zwischen 25 % und 75 % der Erstreckung C des Windelseitenteils in der Längsrichtung beträgt. Ferner sind die jeweiligen Verschlusselemente vorzugsweise im ein- und ausgefalteten Zustand rechteckförmig ausgebildet. Sie sind in der herstellerseitigen nicht aktiven Konfiguration vorzugsweise nach innen auf sich selbst eingefaltet.

In der Gebrauchssituation werden die Windelseitenteile in Überlappung mit einer körperabgewandten Seite, also einer Außenseite, eines Vorderbereichs des Hauptteils gebracht, damit die im Bereich eines jeweiligen in der Windelquerrichtung freien Endes des Windelseitenteils an beiden Windelseitenteilen vorgesehenen Verschlusselemente auf die Außenseite des Vorderbereichs des Hauptteils geschlossen werden können. Hierzu sind die Verschlusselemente und mindestens ein Bereich der Außenseite des Vorderbereichs des Hauptteils als Verschlusssystem ausgebildet. Insbesondere weisen die Verschlusselemente hierzu mechanische Komponenten wie Kletthaken auf, insbesondere auch in Kombination mit haftklebenden Bereichen, vermittels derer die Verschlusselemente lösbar haftend mit der Außenseite des Vorderbereichs des Hauptteils in Eingriff bringbar sind. Hierzu hat es sich als vorteilhaft erwiesen, wenn die Außenseite des Vorderbereichs des Hauptteils zumindest bereichsweise, vorzugsweise vollständig durch einen entsprechend ausgebildeten Vliesstoff gebildet ist. Alternativ ist möglich, ein oder mehrere separate Klettlauschelemente auf der Außenseite des Hauptteils im Vorderbereich vorzusehen, welche als Landezone für die Verschlusselemente der Windelseitenteile dienen.

Weiterhin erweist es sich als vorteilhaft, die Verschlusselemente an die in Querrichtung zweinutzige Windelseitenteilbahn anzufügen. Alternativ ist denkbar, die vierte und fünfte Materialbahn vor dem Anfügen der betreffenden Materialbahn an die Kompositbahn mit Verschlusselementen zu versehen. Als weitere Alternative ist denkbar, Verschlusselemente an die in Querrichtung einnutzigen Windelseitenteilbahnen oder an die Windelseitenteilabschnitte anzufügen. Ebenfalls denkbar ist das Anfügen der Verschlusselemente an die Windelseitenteile nach dem Anfügen der Windelseitenteile an den Hauptteil oder nach der Vereinzelung der Inkontinenzwegwerfwindeln.

Vorzugsweise ist vorgesehen, dass die zweite und dritte Materialbahn durch Trennen einer ersten in ihrer Querrichtung zweinutzigen Materialbahn bereitgestellt werden.

Weiter vorzugsweise werden die vierte und fünfte Materialbahn durch Trennen einer zweiten in ihrer Querrichtung zweinutzigen Materialbahn bereitgestellt.

Nach einer weiteren bevorzugten Ausführungsform werden die erste, vierte und fünfte Materialbahn durch Trennen einer in ihrer Querrichtung dreinutzigen Materialbahn bereitgestellt.

Es hat sich als vorteilhaft erwiesen, die in ihrer jeweiligen Querrichtung einnutzigen, elastisches Material oder Anfügematerial oder Verschlussträgermaterial umfassenden Materialbahnen durch Trennen einer in ihrer jeweiligen Querrichtung zwei- oder dreinutzigen Materialbahn entlang ihrer jeweiligen Längsrichtung bereitzustellen.

Dieser Trennvorgang kann in einer dem Fachmann bekannten Weise "in-line" durchgeführt werden derart, dass die aus dem Trennvorgang erhaltenen in ihrer Querrichtung einnutzigen Materialbahnen direkt anschließend dem weiteren Prozess zur Herstellung der Windelseitenteilbahn zugeführt werden, oder "off-line" derart, dass die einnutzigen Materialbahnen in eine Lagerform gebracht und zu einem späteren Zeitpunkt oder an einer räumlich entfernten Betriebsstätte weiterverarbeitet werden.

Ebenso hat es sich als vorteilhaft erwiesen, die in ihrer jeweiligen Querrichtung einnutzigen Windelseitenteilbahnen durch Trennen einer in ihrer Querrichtung zweinutzigen Windelseitenteilbahn entlang ihrer Längsrichtung bereitzustellen. Dieser Vorgang kann ebenfalls "in-line" oder "off-line" mit Bezug zur Herstellung der Inkontinenzwegwerfwindel erfolgen.

Im Rahmen dieser Erfindung ist unter der Formulierung "Trennen in Längsrichtung" oder "Trennen entlang einer Längsrichtung" bevorzugt eine Trennung entlang einer parallel zur Längserstreckung der betreffenden Bahn verlaufenden geraden Trennlinie zu verstehen. Daneben sind weitere Linienformen denkbar, wie beispielsweise geschwungen, gekrümmt, gebogen, wellenförmig, zackenförmig, stumpfwinklig, spitzwinklig, spitzbogig, flachbogig, oder unregelmäßig, wobei mittels dieser Linienformen Bahnränder erzeugt werden, die nicht geradlinig verlaufen.

Die Trennung kann vollständig, also durchgehend, erfolgen oder diskontinuierlich, gleichsam als Schwächungslinie wie beispielsweise mittels Perforation oder in Form von Materialaussparungen, wobei die Trennung in einem nachfolgenden Verfahrensschritt vollendet wird. Die Trennung wird aus ökonomischen Gründen bevorzugt ohne Anfall von Ausschussmaterial durchgeführt, kann aber, beispielsweise aus ästhetischen Gründen, auch zu einem Aufkommen eines geringen Anteils an zu verwerfendem Material führen.

Sowohl die Trennung von Bahnen in Längsrichtung als auch das Abtrennen von Windelseitenteilabschnitten von einer Windelseitenteilbahn in Querrichtung als auch das Vereinzeln von Inkontinenzwegwerfwindeln in Querrichtung kann mittels unterschiedlichen und dem Fachmann bekannten Verfahren erfolgen, wie beispielsweise Schneiden, Stanzen, Lasern, Fluid-Schneiden (zum Beispiel Wasserstrahl-Schneiden).

Eine bevorzugte Ausführungsform sieht vor, dass die Windelseitenteilabschnitte in ihrer Längsrichtung insbesondere einnutzig ausgebildet sind. Dabei umfasst ein Windelseitenteilabschnitt genau ein Windelseitenteil einer einzigen Inkontinenzwegwerfwindel.

Nach einer weiteren bevorzugten Ausführungsform sind die Windelseitenteilabschnitte in ihrer Längsrichtung zweinutzig ausgebildet, wobei das Trennen der Windelhauptteilbahn durch die Windelseitenteilabschnitte hindurch geführt wird derart, dass ein erster Teilabschnitt eines jeweiligen Windelseitenteilabschnittes ein hinteres Windelseitenteil einer ersten Inkontinenzwegwerfwindel bildet und ein zweiter Teilabschnitt des jeweiligen Windelseitenteilabschnittes ein hinteres Windelseitenteil einer in einer Windellängsrichtung unmittelbar angrenzenden zweiten Inkontinenzwegwerfwindel bildet.

Der erste und der zweite Teilabschnitt sind solchen Falls bevorzugt und im Wesentlichen jeweils gleichartig ausgebildet.

Bevorzugt sind das erste und das zweite elastische Windelseitenteil an jeweils mindestens einer, insbesondere jeweils mindestens zwei, weiter insbesondere jeweils mindestens drei vorzugsweise parallel zur Windellängsrichtung der Inkontinenzwegwerfwindel sich erstreckenden Faltlinien FW1 auf sich selbst gefaltet und insbesondere in dieser Konfiguration lösbar fixiert.

Eine derartige Faltung der Windelseitenteile erweist sich als vorteilhaft zum einen beim Verpacken der Inkontinenzwegwerfwindel, und zum anderen beim Anlegen der Inkontinenzwegwerfwindel durch den Verwender. Die so erhaltene gefaltete Konfiguration kann herstellerseitig vorzugsweise fixiert sein, etwa durch einzelne Fügestellen, insbesondere Klebe- oder Schweiß-, insbesondere Ultraschallschweißstellen, die gleichwohl zum Entfalten der Windelseitenteile durch den Benutzer verhältnismäßig leicht, insbesondere in einem Zug, manuell lösbar sind. In diesem Fall erweist sich ein einziges in der Windellängsrichtung vorzugsweise ungefähr mittig an den Windelseitenteilen positioniertes Verschlusselement als vorteilhaft, wobei weiter vorzugsweise dann die Fügestellen das eingefaltete Verschlusselement nicht erfassen, sondern in der Windellängsrichtung außerhalb des Verschlusselements angeordnet sind. Wenn die gegeneinander anliegenden Teilbereiche um das wenigstens eine nach innen eingeschlagene Verschlusselement herum oder in der Windellängsrichtung oberhalb oder unterhalb des eingeschlagenen Verschlusselements durch die vorgenannten Maßnahmen lösbar fixiert sind, so bildet das eingeschlagene Verschlusselement einen gut ergreifbaren Anfassbereich zum Entfalten des jeweiligen Windelseitenteils.

In vorteilhafter Weiterentwicklung der oben beschriebenen Ausführung erweist es sich als vorteilhaft, wenn die Windelseitenteile herstellerseitig um wenigstens zwei, insbesondere wenigstens drei in der Windellängsrichtung verlaufende Faltlinien FW1 insbesondere Z-förmig auf sich selbst gefaltet sind und durch diese Faltlinien FW1 aufeinander gefaltete Teilbereiche der Windelseitenteile definiert und begrenzt werden, und weiter vorzugsweise dass ein in der Querrichtung außen liegender Teilbereich im Wesentlichen undehnbar ausgebildet ist. Hierdurch werden insbesondere die Ergreifbarkeit und die Entfaltbarkeit sowohl des Windelseitenteils als auch des eingefalteten Verschlusselements verbessert.

Weiter erweist sich als vorteilhaft, wenn ein an den außen liegenden Teilbereich nach innen anschließender Teilbereich ausgehend von einer dem in Windelquerrichtung freien Ende des Windelseitenteils am nächsten gelegenen in Windellängsrichtung verlaufenden Faltlinie mit wenigstens 40 % seiner Fläche undehnbar ausgebildet ist. Zur Bestimmung dieser Fläche wird auf WO 2017/140604 A1 verwiesen. Hierdurch wird erreicht, dass der in der Windelquerrichtung außen liegende Teilbereich und der daran nach innen anschließende Teilbereich über eine sehr große undehnbare Fläche (von wenigstens 40 % der Fläche des letztgenannten Teilbereichs), die demzufolge frei von elastischen oder elastifizierenden Elementen ist, flächenhaft aneinander anliegen.

In vorteilhafter Weiterentwicklung dieser Ausführung beträgt eine Erstreckung E eines jeweiligen undehnbaren Bereichs des an den außen liegenden Teilbereich nach innen anschließenden Teilbereichs ausgehend von der äußeren in der Windellängsrichtung verlaufenden Faltlinie in Windelquerrichtung bis zum Beginn eines dehnbaren Bereichs vorzugsweise mindestens 15 mm, insbesondere mindestens 20 mm, weiter insbesondere mindestens 25 mm, weiter vorzugsweise mindestens 30 mm, vorzugsweise jedoch höchstens 100 mm, weiter vorzugsweise höchstens 85 mm, weiter vorzugsweise höchstens 70 mm.

Für die eingefaltete Konfiguration der Windelseitenteile gilt vorzugsweise, dass eine Erstreckung A der auf sich selbst gefalteten Windelseitenteile in der Windelquerrichtung über den jeweiligen hinteren seitlichen Längsrand hinaus und eine Erstreckung C der auf sich selbst gefalteten Windelseitenteile in der Windellängsrichtung der Inkontinenzzwegwerfwindel derart bemessen ist, dass das Verhältnis der Erstreckungen A/C zueinander 0,5 < A/C <1 beträgt.

Zur Bildung der Faltlinien FW1 der Windelseitenteile der Inkontinenzzwegwerfwindel hat es sich als vorteilhaft erwiesen, wenn die zweinutzige Windelseitenteilbahn beidseitig um jeweils mindestens eine, insbesondere um jeweils mindestens zwei, weiter insbesondere um jeweils mindestens drei zur Längsrichtung der zweinutzigen Windelseitenteilbahn parallelen Faltlinien FW2 auf sich selbst gefaltet und insbesondere in dieser Konfiguration lösbar fixiert wird.

Das Längsfalten der Windelseitenteilbahn kann aber nach einer Alternative auch nach dem Trennen der zweinutzigen Windelseitenteilbahn zur Bildung von einnutzigen Windelseitenteilbahnen erfolgen. Solchen Falls werden die einnutzigen Windelseitenteilbahnen um jeweils mindestens eine, insbesondere um jeweils mindestens zwei, weiter insbesondere um jeweils mindestens drei zu einer Längsrichtung der jeweiligen einnutzigen Windelseitenteilbahn parallelen Faltlinien FW3 oder FW4 auf sich selbst gefaltet und insbesondere in dieser Konfiguration lösbar fixiert.

Die Faltung der Windelseitenteilabschnitte um die Faltlinien FW2, FW3 oder FW4 und insbesondere der in ihrer jeweiligen Querrichtung zweinutzigen oder einnutzigen Windelseitenteilbahn insbesondere in Kombination mit der Fixierung der gefalteten Konfiguration mit oben genannten Methoden bewirkt, dass die Windelseitenteilabschnitte bereits vor ihrem Anfügen an den Windelhauptteil vorgefaltet sind, und hat den Vorteil, dass die vorgefalteten Windelseitenteilabschnitte steifer und in ihrer jeweiligen Querrichtung kürzer sind als ungefaltete Windelseitenteilabschnitte und sich daher insbesondere in schnell laufenden Maschinen einfacher verarbeiten lassen. Daneben ist eine Ausführungsform denkbar, bei der die Faltung der Windelseitenteile erst nach dem Anfügen an den Windelhauptteil erfolgt.

Vorzugsweise werden die Windelseitenteilabschnitte auf die Windelhauptteilbahn und dabei insbesondere auf eine Oberseite der Windelhauptteilbahn gefaltet. Solchen Falls kann die jeweilige Faltlinie um die ein Windelseitenteilabschnitt auf die Windelhauptteilbahn gefaltet wird innerhalb der Windelhauptteilbahn oder innerhalb des sich über die Windelhauptteilbahn hinaus erstreckenden Bereiches des Windelseitenteilabschnittes, also außerhalb der Windelhauptteilbahn verlaufen. Die Oberseite der Windelhauptteilbahn kann die körperabgewandte, insbesondere jedoch die körperzugewandte Seite der Windelhauptteilbahn sein. Es ist alternativ denkbar, dass erst nach der Vereinzelung der Inkontinenzwegwerfwindel die durch die Windelseitenteilabschnitte gebildeten Windelseitenteile auf den Windelhauptteil der Inkontinenzzwegwerfwindel gefaltet werden.

Vor der herstellerseitigen Verpackung der Inkontinenzzwegwerfwindel wird vorzugsweise der Hauptteil herstellerseitig mitsamt den auf sich selbst gefalteten Windelseitenteilen und/oder den auf den Hauptteil gefalteten Windelseitenteilen um eine erste und eine zweite jeweils in der Windellängsrichtung verlaufende Hauptteil-Faltlinie nach innen auf sich selbst gefaltet, vorzugsweise derart, dass die beidseitigen Windelseitenteile in Dickenrichtung, also orthogonal zu einer die Windellängsrichtung und die Windelquerrichtung einschließenden Ebene, in wenigstens teilweiser Überlappung zueinander zu liegen kommen. Weiter vorzugsweise wird vor der herstellerseitigen Verpackung der Inkontinenzzwegwerfwindel der Hauptteil herstellerseitig mitsamt den auf sich selbst gefalteten Windelseitenteilen und vorzugsweise im Anschluss an die zuvor beschriebene Faltung um in der Windellängsrichtung verlaufende Hauptteil-Faltlinien zusätzlich vorzugsweise um mindestens eine oder zwei in der Windelquerrichtung verlaufende Hauptteil-Faltlinien zu einer Innenseite, also einer im Gebrauchszustand körperzugewandten Seite des Hauptteils auf sich selbst gefaltet, derart, dass eine körperzugewandte Seite des Hauptteils zumindest bereichsweise direkt oder mittelbar auf sich selbst zu liegen kommt.

In einer bevorzugten Ausführungsform der vorliegenden Erfindung werden die vereinzelten Inkontinenzzwegwerfwindeln um mindestens eine, insbesondere zwei in einer Windelquerrichtung erstreckte Faltlinien FQ gefaltet.

An dieser Stelle sei ausgeführt, dass die Bahnen, mithin die erste, zweite, dritte, vierte und fünfte Materialbahn und die Kompositbahn, jeweils beidseitige Bahnränder aufweisen, wobei sich an die Bahnränder jeweils ein sich flächig in Richtung des jeweils anderen Bahnrandes der jeweiligen Bahn erstreckender Bahnrandbereich anschließt.

Beim Zusammenfügen zweier Bahnen werden diese Bahnen jeweils entlang ihrer jeweiligen Längsrichtung, vorzugsweise parallel zueinander gefördert, derart, dass die beiden Bahnen vorzugsweise flach nebeneinander zu liegen kommen.

Das Zusammenfügen zweier Bahnen kann mittels dem Fachmann an sich bekannter Fügeverfahren, insbesondere durch Thermoschweißen, Ultraschallschweißen, Nähen, Siegeln oder Kleben, erfolgen, wobei die beiden Bahnen bevorzugt unlösbar miteinander verbunden werden.

Die beiden zu verbindenden Bahnen können mit ihren jeweils in Richtung der anderen Bahn weisenden Bahnrandbereichen aneinanderstoßend, mithin "auf Stoß", mit oder ohne den Stoß der beiden Bahnen überbrückende zusätzliche Materialbahnen gefügt werden.

Als besonders vorteilhaft hat es sich erwiesen, die beiden zu verbindenden Bahnen mit ihren jeweils in Richtung der anderen Bahn weisenden Bahnrandbereichen überlappend zu führen und die Bahnen in einem dabei gebildeten Überlappungs- oder Fügebereich durch oben genannte und dem Fachmann an sich bekannte Fügeverfahren unlösbar zu verbinden.

In einer bevorzugten Ausführungsform weist ein Quotient Q1 aus der Breite B1 und der Summe der Breiten B2 und B3 berechnet als: B1/(B2+B3) einen Wert von 0,2 bis 1,5, insbesondere einen Wert von 0,25 bis 1,2, insbesondere einen Wert von 0,25 bis 1,0, insbesondere einen Wert von 0,3 bis 0,9, insbesondere einen Wert von 0,4 bis 0,8 auf und/oder ein Quotient Q2 aus der Breite B1 und der Summe der Breiten B4 und B5 berechnet als: B1/(B4+B5) weist einen Wert von 0,15 bis 1,5, insbesondere einen Wert von 0,2 bis 1,2, insbesondere einen Wert von 0,2 bis 1,0, insbesondere einen Wert von 0,25 bis 0,7, insbesondere einen Wert von 0,3 bis 0,5 auf.

Weiter bevorzugt gilt B1≤(B2+B3) und/oder es gilt B1≤(B4+B5).

Die Breite B1 der ersten Materialbahn beträgt vorzugsweise 50 - 150 mm, insbesondere 70 - 130 mm, weiter insbesondere 90 - 110 mm.

Die Breite B2 der zweiten Materialbahn und/oder die Breite B3 der dritten Materialbahn beträgt vorzugsweise jeweils 20 - 130 mm, insbesondere 30 - 120 mm, weiter insbesondere 40 - 100 mm.

Die Breite B4 der vierten Materialbahn und/oder die Breite B5 der fünften Materialbahn beträgt vorzugsweise jeweils 50 - 200 mm, insbesondere 60 - 190 mm, weiter insbesondere 70 - 180 mm.

In dem Fall, dass beim Aneinanderfügen der ersten Materialbahn und der zweiten und/oder dritten Materialbahn die in Richtung der jeweils benachbarten Bahn weisenden Bahnrandbereiche überlappend geführt werden, beträgt eine Breite B6 des Überlappungsbereichs der ersten Materialbahn mit der zweiten Materialbahn und/oder eine Breite B7 des Überlappungsbereichs der ersten Materialbahn mit der dritten Materialbahn jeweils vorzugsweise 3 - 17 mm, weiter insbesondere 5 - 15 mm, weiter insbesondere 6 - 13 mm.

In dem Fall, dass beim Aneinanderfügen der Kompositbahn und der vierten und/oder fünften Materialbahn die in Richtung der jeweils benachbarten Bahn weisenden Bahnrandbereiche überlappend geführt werden, beträgt eine Breite B8 des Überlappungsbereichs der Kompositbahn mit der vierten Materialbahn und/oder eine Breite B9 des Überlappungsbereichs der Kompositbahn mit der fünften Materialbahn jeweils vorzugsweise 3 - 17 mm, weiter insbesondere 5 - 15 mm, weiter insbesondere 6 - 13 mm.

In einer bevorzugten Ausführungsform beträgt eine Breite B11 der Kompositbahn 150 - 400 mm, insbesondere 170 - 350 mm, weiter insbesondere 200 - 300 mm.

Eine Breite B10 der in ihrer Querrichtung zweinutzigen Windelseitenteilbahn beträgt vorzugsweise 300 - 650 mm, insbesondere 320 - 630 mm, weiter insbesondere 340 - 610 mm.

Ein Quotient Q3 aus der Breite B11 (Zähler) und der Breite B10 (Nenner) weist vorzugsweise einen Wert von 0,25 - 0,8, weiter insbesondere 0,3 - 0,75, weiter insbesondere 0,4 - 0,7 auf.

Weiter vorzugsweise beträgt die Längserstreckung der in ihrer Längsrichtung einnutzigen Windelseitenteilabschnitte und/oder der in Windellängsrichtung einnutzigen Windelseitenteile jeweils 110 - 170 mm, insbesondere 120 - 160 mm, weiter insbesondere 130 - 150 mm.

Die Breite B1 der ersten Materialbahn, die Breite B2 der zweiten Materialbahn, die Breite B3 der dritten Materialbahn, die Breite B4 der vierten Materialbahn, die Breite B5 der fünften Materialbahn, die Breite B11 der Kompositbahn und die Breite B10 der in ihrer Querrichtung zweinutzigen Windelseitenteilbahn werden jeweils in Querrichtung der jeweiligen Bahn, also entlang einer Quererstreckung, die mit einem Winkel von 90° zur Längsrichtung der jeweiligen Bahn verläuft, im plan ausgebreiteten, nicht gefalteten und nicht gedehnten Zustand in mm gemessen.

Der Begriff "Breite" ist im Rahmen der vorliegenden Erfindung als maximale Erstreckung in Querrichtung der jeweiligen Bahn zu verstehen.

Ebenso werden die jeweiligen Breiten B6, B7, B8 oder B9 der Überlappungs- oder Fügebereiche und die nachfolgend näher beschriebenen Breiten B2e und B3e der effektiven elastischen Bereiche der zweinutzigen Windelseitenteilbahn in Querrichtung der zweinutzigen Windelseitenteilbahn im plan ausgebreiteten, nicht gefalteten Zustand gemessen.

Windellängs- oder Windelquererstreckungen und Breiten der Inkontinenzwegwerfwindel und/oder ihrer ersten oder zweiten elastischen Windelseitenteile, mithin die Erstreckung E, weiter der Abstand D, weiter nachfolgend näher beschriebene Breiten B2e.1, B1.1 und B4.1, werden im plan ausgebreiteten, nicht gefalteten und nicht gedehnten Zustand der Inkontinenzwegwerfwindel und/oder ihrer ersten und zweiten elastischen Windelseitenteile, also bezüglich der Dehnung der elastischen Windelseitenteile im Zustand der ersten In-die-Hand-Nahme durch den Benutzer, jedoch nach dem vollständigen Auffalten der herstellerseitigen Faltungen der Inkontinenzwegwerfwindel und/oder ihrer elastischen Windelseitenteile in mm gemessen.

Die Erstreckungen A und C der ersten und zweiten elastischen Windelseitenteile werden im plan ausgebreiteten und nicht gefalteten Zustand der Inkontinenzwegwerfwindel in mm gemessen, wobei jedoch das erste oder zweite elastische Windelseitenteil im gefalteten, nicht gedehnten und plan ausgebreiteten Zustand verbleibt.

An dieser Stelle sei ausgeführt, dass die Formulierung "nicht gedehnt" so zu verstehen ist, dass das Entfalten und flache Ausbreiten der elastischen Windelseitenteile ohne die durch den Benutzer beim Anlegen der Inkontinenzwegwerfwindel typischerweise ausgeübten Zugkräfte erfolgt, so dass das Material der nicht gedehnten elastischen Windelseitenteile im herstellerseitig vor der Benutzung vorgesehenen relaxierten Zustand verbleibt.

Umfasst ist im Rahmen der vorliegenden Erfindung auch eine Inkontinenzwegwerfwindel hergestellt oder herstellbar nach sämtlichen vor- oder nachstehenden Ausführungsformen. Besonders bevorzugt ist die Inkontinenzwegwerfwindel als sogenannte T-Form Windel ausgeführt. Insbesondere sind an den Vorderbereich der Inkontinenzwegwerfwindel keine Windelseitenteile angefügt.

Der Hauptteil der Inkontinenzwegwerfwindel umfasst vorzugsweise ein zumindest bereichsweise flüssigkeitsdurchlässiges Topsheet und ein vorzugsweise atmungsaktives und vorzugsweise zumindest bereichsweise flüssigkeitsundurchlässiges Backsheet, welche den Saugkörper sandwichartig umgeben. Weiterhin kann die Inkontinenzwegwerfwindel auf der körperzugewandten Seite des Schrittbereichs beidseits jeweils eine seitliche Auslaufsperre bildende und/oder beidseits entlang einer Längserstreckung des Saugkörpers verlaufende Cuffelemente aufweisen. Die Cuffelemente umfassen vorzugsweise ein Vliesmaterial ("Cuffvlies"), insbesondere ein hydrophobes Vliesmaterial, oder sind daraus gebildet. Vorzugsweise erstrecken sich Topsheet und/oder Backsheet und/oder Cuffvlies zumindest in Windelquerrichtung, vorzugsweise auch in Windellängsrichtung über die Konturränder des Saugkörpers hinaus, um einen entsprechenden Überhang zu bilden. In dem Überhang sind Backsheet und Topsheet und/oder Cuffvlies vorzugsweise zumindest bereichsweise miteinander verbunden, insbesondere durch an sich bekannte Fügeverfahren wie Schweißen, Siegeln, Nähen oder Kleben.

In vorteilhafter Weise weist der Hauptteil in dem Schrittbereich beidseitig an einen jeweiligen Längsrand des Schrittbereichs angrenzend je einen in Windellängsrichtung der Inkontinenzwegwerfwindel erstreckten elastischen oder elastifizierten Abschnitt, mithin einen elastischen oder elastifizierten Beinöffnungsabschnitt auf. "In Windellängsrichtung" bedeutet hierbei, dass der elastische oder elastifizierte Beinöffnungsabschnitt zumindest eine Komponente in Windellängsrichtung aufweist, mithin auch schräg oder gekrümmt zur Windellängsrichtung verlaufen kann.

Vorzugsweise sind hierzu dem Fachmann an sich bekannte elastische Fäden (Lycra® o.Ä.) in vorgespanntem Zustand an den Hauptteil bildenden Materialien fixiert, vorzugsweise an Top- und/oder Backsheet des Hauptteils, insbesondere in einem Bereich, in dem Top- und/oder Backsheet und/oder Cuffvlies einen Überhang außerhalb der Konturränder des Saugkörpers bilden. Ein elastischer oder elastifizierter Beinöffnungsabschnitt kann nach einer Variante auch durch flachmaterial- oder bandförmige Materialien wie elastische Bänder, Folien, Vliesstoffe oder Schaumstoffe gebildet sein.

Der Saugkörper ist geeignet und dazu bestimmt Körperausscheidungen, insbesondere Körperflüssigkeiten, insbesondere Urin aufzunehmen und dauerhaft zu speichern. Der Saugkörper kann hierzu vorteilhaft superabsorbierendes Polymermaterial (SAP) enthalten, insbesondere zu 5 - 100 Gewichtsprozent, vorzugsweise zu 10 - 95 Gewichtsprozent, weiter vorzugsweise zu 15 - 90 Gewichtsprozent, ganz besonders bevorzugt zu 20 - 80 Gewichtsprozent. Typischerweise kann das SAP-Material zumindest das 15-fache, insbesondere das 20-fache seines Gewichts an 0,9 gewichtsprozentiger Kochsalzlösung absorbieren (gemessen nach NWSP 242.0.R2(15)).

Das SAP-Material kann beispielsweise partikelförmig oder faserförmig oder blatt- oder schaumförmig ausgebildet sein.

Der Saugkörper kann weitere Materialien, wie Zellstofffasern (wood pulp) oder Kunststofffasern enthalten. Denkbar ist weiterhin, den Saugkörper durch Anordnung von ein oder mehreren Schichten verschiedenen Materials, insbesondere aus Vliesstoff auszubilden.

Der Saugkörper ist vorzugsweise integraler Bestandteil des Hauptteils und solchen Falls herstellerseitig unlösbar mit den weiteren Komponenten des Hauptteils verbunden. Solchen Falls ist der Saugkörper vorzugsweise unlösbar mit einem Topsheet und/oder mit einem Backsheet des Hauptteils verbunden.

Zur Prüfung, ob ein Material als "elastisch" in der jeweiligen Richtung einzustufen ist, ist im Rahmen der vorliegenden Erfindung folgende Prüfung der bleibenden Dehnung (Permanent Set) vorzunehmen:
Für die Durchführung der Prüfungen wurde eine Zugprüfmaschine Typ Shimadzu AG-Xplus, gemäß DIN EN ISO 7500-01:2004-11, mit unterer fester Klemmbacke und oberer beweglicher Klemmbacke verwendet. Vorzugsweise verfügt die Testvorrichtung über eine Software, welche den Prüfzyklus steuert und ein automatisiertes Aufzeichnen der Kraftwerte sicherstellt.

Bei der Durchführung der Prüfmethode wird der zu prüfende rechteckige Materialabschnitt idealerweise mit einer Breite von 25 mm und einer Länge von 60 mm (die Länge entspricht der Zugrichtung) als Prüfling bereitgestellt. Hierzu wird der Prüfling zum Beispiel aus einer entsprechenden elastischen Komponente der Inkontinenzwegwerfwindel oder einer jeweiligen Materialbahn herausgestanzt, wobei die Zugrichtung des Prüflings der Windelquerrichtung der Inkontinenzwegwerfwindel bzw. der Querrichtung der Materialbahn entspricht.

Idealerweise wird der 25 mm breite und 60 mm lange (Zugrichtung) Prüfling in die Klemmen einer Zugprüfmaschine mit Klemmbackenabstand von 40 mm eingespannt, eine Vorspannung von 0,05 N angefahren und eine zyklische Bewegung (Be- und Entlastung, Prüfgeschwindigkeit 500 mm/min), ohne Haltezeit im oberen Umkehrpunkt, zwischen L0 (Länge des Prüfabschnitts bei Vorspannung von 0,05 N) und einer Maximaldehnung von 60 % durchgeführt. Bei Entlastung wird die Endlänge L1 ebenfalls bei 0,05 N notiert.

Als bleibende Dehnung (permanent set) PS wird berechnet:

$$PS = ((L1-L0):L0) \times 100 \ [\%].$$

Als elastisch in der jeweiligen Richtung wird ein Material dann angesehen, wenn die bleibende Dehnung weniger als 25 % beträgt.

Sollte kein 25 mm breiter Abschnitt der zu prüfenden Komponente zur Verfügung stehen, wird ein entsprechend schmalerer Abschnitt verwendet. Sollte kein 60 mm langer Abschnitt der elastischen Komponente zur Verfügung stehen, wird ein entsprechend kürzerer Abschnitt verwendet, wobei der Klemmbackenabstand, mithin die effektive Länge des Prüfabschnitts ggf. auf bis zu 15 mm zu verringern ist. Es ist sicherzustellen, dass der Abschnitt fest in die Klemmbacken eingespannt wird.

Zur Prüfung, ob ein Material oder eine Komponente als "undehnbar" in der jeweiligen Richtung einzustufen ist, ist im Rahmen der vorliegenden Erfindung folgende Prüfung vorzunehmen:

Für die Durchführung der Prüfungen wurde eine Zugprüfmaschine Typ Shimadzu AG-Xplus, gemäß DIN EN ISO 7500-01:2004-11, mit unterer fester Klemmbacke und oberer beweglicher Klemmbacke verwendet. Vorzugsweise verfügt die Testvorrichtung über eine Software, welche den Prüfzyklus steuert und ein automatisiertes Aufzeichnen der Kraftwerte sicherstellt.

Bei der Durchführung der Prüfmethode wird der zu prüfende rechteckige Materialabschnitt idealerweise mit einer Breite von 25 mm und einer Länge von 60 mm (die Länge entspricht der Zugrichtung) als Prüfling bereitgestellt. Hierzu wird der Prüfling zum Beispiel aus einer entsprechenden Komponente der Inkontinenzwegwerfwindel oder einer jeweiligen Materialbahn herausgestanzt, wobei die Zugrichtung des Prüflings der Windelquerrichtung der Inkontinenzwegwerfwindel bzw. der Querrichtung der Materialbahn entspricht.

Idealerweise wird ein 25 mm breiter und 60 mm langer (Zugrichtung) Prüfling des Materials unter Vorspannung von 0,05 N in die Klemmen der Zugprüfmaschine mit Klemmbackenabstand von 40 mm eingespannt und der Prüfling bis zu einer Kraft von 5 N gedehnt (belastet, Prüfgeschwindigkeit 500 mm/min). Sollte die Dehnung bei der Kraft von 5 N weniger als 30 % betragen, wird das Material im Rahmen der vorliegenden Erfindung als undehnbar bezeichnet. Im Falle, dass der Prüfling bei der Ausführung des vorstehenden Tests bricht, bevor die maximale Kraft von 5 N erreicht ist, wird das Material im Rahmen der vorliegenden Erfindung ebenfalls als "undehnbar" in der jeweiligen Richtung eingestuft.

Sollte kein 25 mm breiter Abschnitt der zu prüfenden Komponente zur Verfügung stehen, wird ein entsprechend schmalerer Abschnitt verwendet. Solchen Falls wird anstelle der Kraft von 5 N der einzustellende Kraftwert errechnet, indem die Belastung von 5 N um den Faktor f1 reduziert wird, welcher sich ergibt aus f1=25/x, wobei x die Breite des Prüflings gemessen in mm ist. Beispiel: ein 12,5 mm breiter Prüfling würde einer Kraft von 2,5 N ausgesetzt werden.

Sollte kein 60 mm langer Abschnitt der elastischen Komponente zur Verfügung stehen, wird ein entsprechend kürzerer Abschnitt verwendet, wobei der Klemmbackenabstand, mithin die effektive Länge des Prüfabschnitts ggf. auf bis zu 15 mm zu verringern ist. Es ist sicherzustellen, dass der Abschnitt fest in die Klemmbacken eingespannt wird.

Weitere Merkmale, Einzelheiten und Vorteile der Erfindung ergeben sich aus den beigefügten Patentansprüchen und aus der zeichnerischen Darstellung und nachfolgenden Beschreibung bevorzugter Ausführungsformen der Erfindung. In der Zeichnung zeigt:

**Figur 1**
eine schematische Darstellung der Prozessschritte eines Verfahrens zur Herstellung von Inkontinenzwegwerfwindeln;

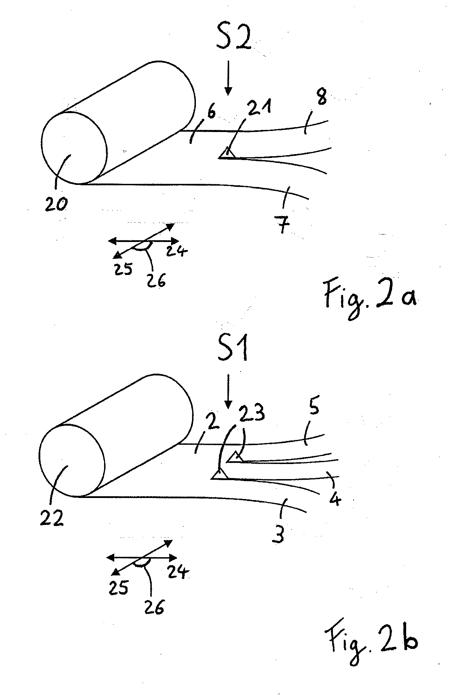

**Figur 2a und 2b**
schematisch, das Trennen zwei- (Figur 2a) und dreinutziger (Figur 2b) Materialbahnen in Längsrichtung;

**Figur 3a, 3b und 3c**
schematische Darstellungen in Draufsicht

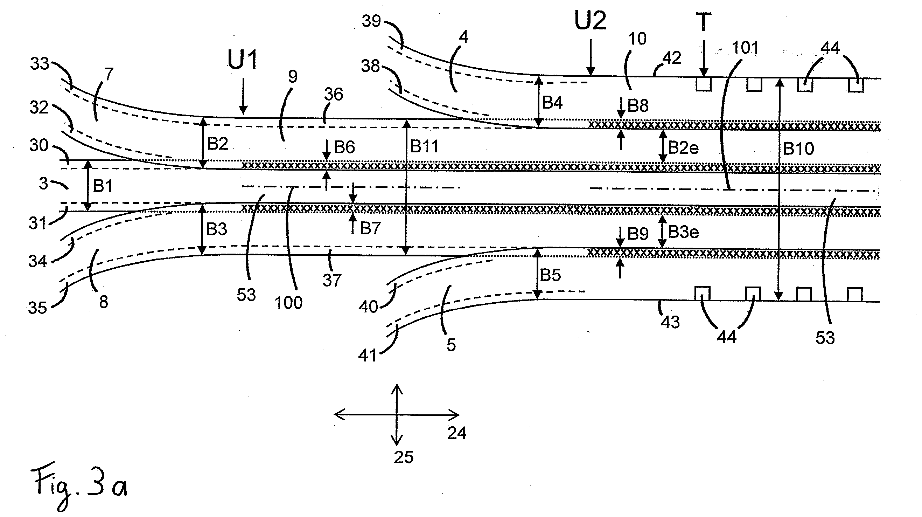

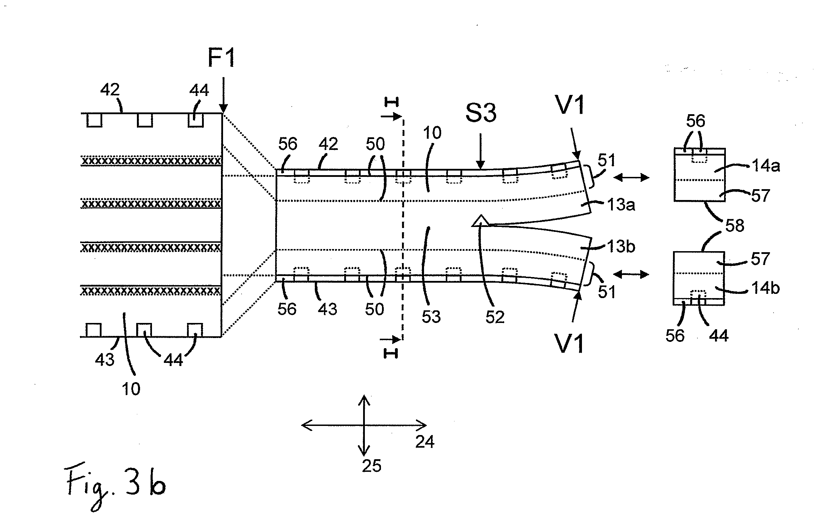

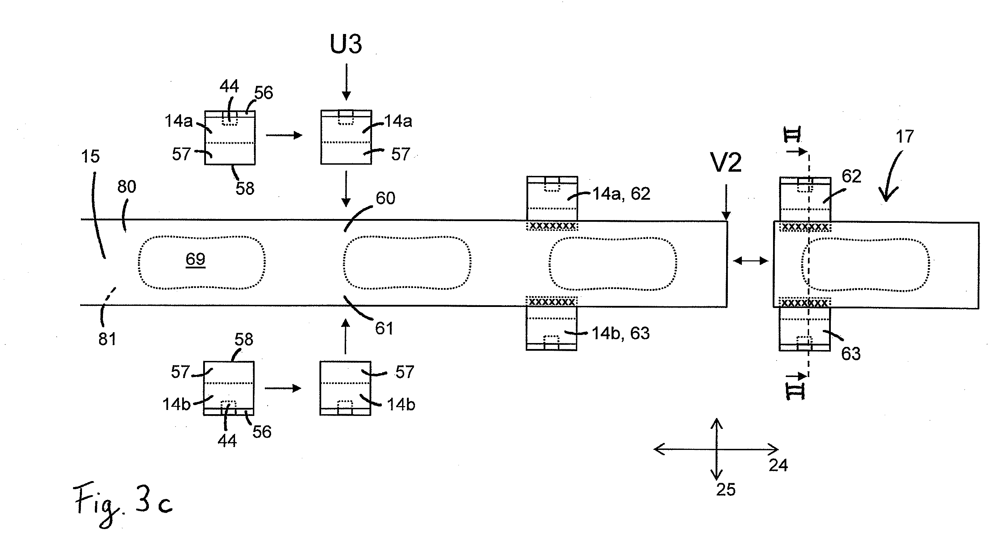

- der Anfügeschritte zur Bildung einer zweinutzigen Windelseitenteilbahn (Figur 3a),
- der Faltung einer zweinutzigen Windelseitenteilbahn, Längstrennung und Vereinzelung von Windelseitenteilabschnitten (Figur 3b),
- des Anfügens von Windelseitenteilabschnitten an eine Windelhauptteilbahn und Vereinzelung von Inkontinenzwegwerfwindeln (Figur 3c);

**Figur 4**
schematische Darstellung in Draufsicht der Längstrennung einer zweinutzigen Windelseitenteilbahn und Faltung einnutziger Windelseitenteilbahnen auf sich selbst und Vereinzelung von Windelseitenteilabschnitten;

**Figur 5**
schematisch, einen Querschnitt durch eine in Querrichtung zweinutzige Windelseitenteilbahn;

**Figur 6**
schematisch, eine Draufsicht auf eine Inkontinenzwegwerfwindel im eben ausgebreiteten, jedoch nicht gedehnten Zustand mit einem gefalteten und einem nicht gefalteten Windelseitenteil;

**Figur 7**
schematisch, eine Inkontinenzwegwerfwindel im angelegten Zustand;

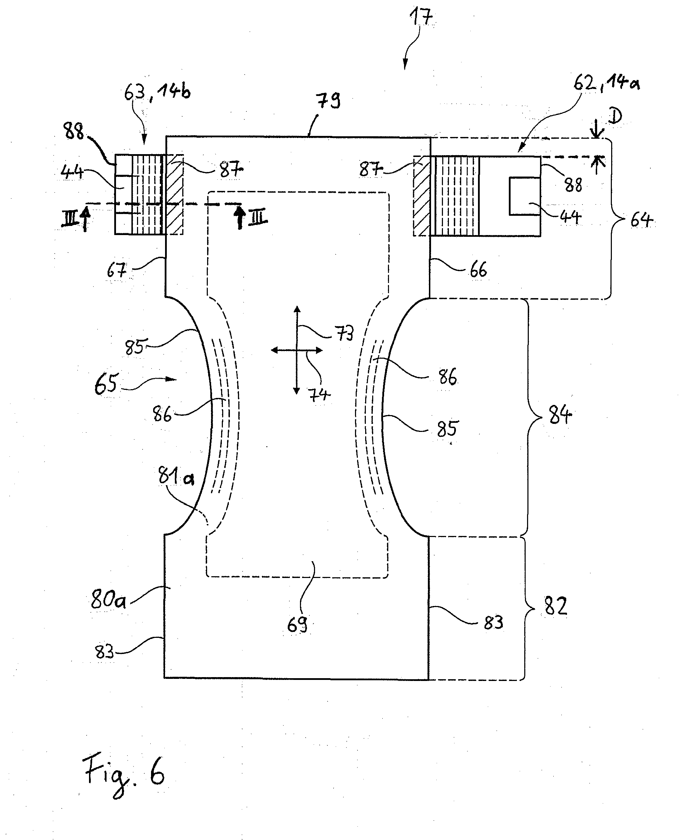

**Figur 8**
schematisch, eine vergrößerte Darstellung der Inkontinenzwegwerfwindel nach Figur 6 im Bereich eines hinteren elastischen Windelseitenteils in eben ausgebreitetem, jedoch nicht gedehntem Zustand;

**Figur 9**

schematisch, eine vergrößerte Darstellung der Inkontinenzwegwerfwindel nach Figur 6 im Bereich eines hinteren elastischen Windelseitenteils in auf sich selbst gefalteter Konfiguration;

**Figur 10a**

schematisch, eine Schnittansicht eines Windelseitenteils mit Z-Faltung mit Schnittebene III-III Figur 6;

**Figur 10b**

schematisch, eine Schnittansicht einer W-Faltung eines Windelseitenteils;

**Figur 10c**

schematisch, eine Schnittansicht einer C-Faltung eines Windelseitenteils;

**Figur 11**

schematisch, eine Schnittansicht mit Schnittebene I-I Figur 3b;

**Figur 12a**

schematisch, eine Schnittansicht einer Inkontinenzwegwerfwindel mit Schnittebene II-II Figur 3c;

**Figur 12b**

schematisch, eine Schnittansicht einer Inkontinenzwegwerfwindel mit einem Topsheet gebildet aus drei Flachmaterialbahnen.

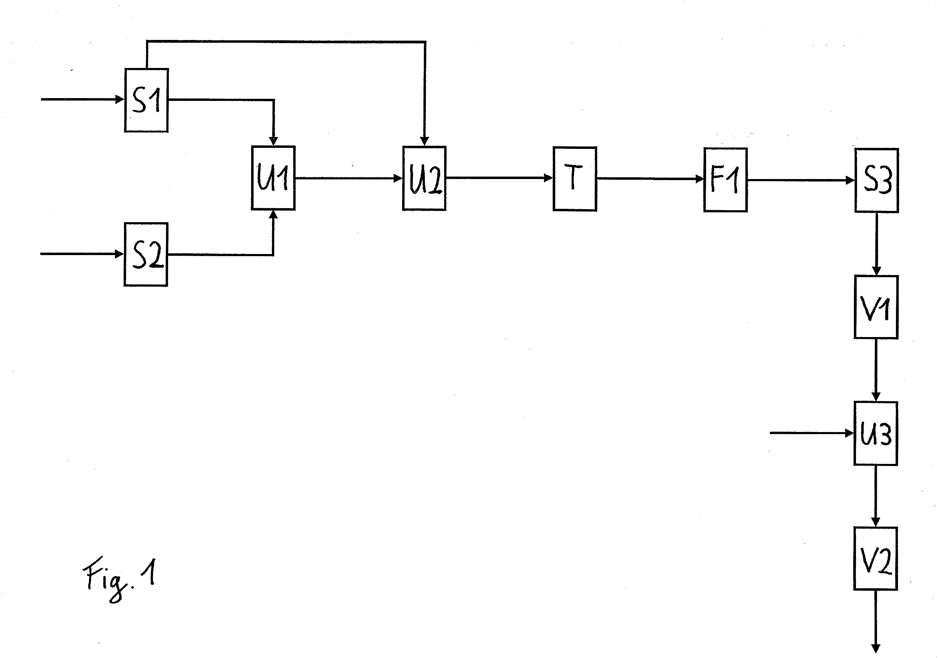

Mit Bezug zu Figuren 1, 2, 3a, 3b, 3c wird nachfolgend beispielhaft ein erfindungsgemäßes Verfahren zur Herstellung von Inkontinenzwegwerfwindeln 17 beschrieben. Das beispielhaft dargestellte Verfahren umfasst mehrere Schritte, mithin Trenn-, Füge-, Falt- und Vereinzelungsschritte sowie das Anbringen von Verschlusselementen, die in nachfolgenden Figuren jeweils näher beschrieben sind. In einer bevorzugten Ausführungsform wird in einer ersten Schneidestation S1 eine in ihrer Querrichtung 25 dreinutzige Materialbahn 2, die im Wesentlichen in ihrer Querrichtung 25 undehnbares Material umfasst, nämlich Anfügematerial oder Verschlussträgermaterial, in ihrer Längsrichtung 24 in drei (mit Bezugszeichen 3, 4 und 5 versehene) jeweils in ihrer Querrichtung einnutzige Materialbahnen, nämlich die erste Materialbahn 3, die vierte Materialbahn 4 und die fünfte Materialbahn 5 getrennt. Eine in ihrer Querrichtung 25 zweinutzige Materialbahn 6, die in Querrichtung 25 zumindest bereichsweise elastisches Material umfasst, wird in einer zweiten Schneidestation S2 in Längsrichtung in zwei jeweils in ihrer Querrichtung einnutzige Materialbahnen, nämlich die zweite Materialbahn 7 und die dritte Materialbahn 8 getrennt, wobei die zweite Materialbahn 7 und die dritte Materialbahn 8 jeweils in ihrer Querrichtung zumindest bereichsweise elastisches Material umfassen. Die Materialbahnen werden nachfolgend einander zugeführt und aneinander angefügt, und zwar derart, dass in einer ersten Fügestation U1 die erste Materialbahn 3, die im Wesentlichen Anfügematerial umfasst (oder daraus besteht), und die jeweils zumindest bereichsweise in Querrichtung 25 elastisches Material umfassenden zweite Materialbahn 7 und dritte Materialbahn 8 derart aneinandergefügt werden, dass sie zusammen eine Kompositbahn 9 bilden, bei der das Anfügematerial der ersten Materialbahn 3 in Querrichtung 25 zwischen der zweiten Materialbahn 7 und der dritten Materialbahn 8 angeordnet ist, bei der also in Querrichtung 25 das Anfügematerial der ersten Materialbahn 3 einen zentralen Bereich 53 bildet. In einer weiteren Fügestation U2 werden die vierte Materialbahn 4 und die fünfte Materialbahn 5, die jeweils im Wesentlichen in Querrichtung 25 undehnbares Verschlussträgermaterial umfassen (oder daraus bestehen), wie nachfolgend näher beschrieben an die Kompositbahn 9 angefügt derart, dass die Kompositbahn 9 in Querrichtung 25 zwischen der vierten Materialbahn 4 und der fünften Materialbahn 5 angeordnet wird und die Kompositbahn 9 gemeinsam mit der vierten Materialbahn 4 und der fünften Materialbahn 5 eine in Querrichtung 25 zweinutzige Windelseitenteilbahn 10 bildet. In einer Taper-Station T werden Verschlusselemente ("Tapes") 44 beidseits an das Verschlussträgermaterial der zweinutzigen Windelseitenteilbahn 10 angefügt. Die die Verschlusselemente 44 umfassende zweinutzige Windelseitenteilbahn 10 wird einer Faltstation F1 zugeführt, in der die zweinutzige Windelseitenteilbahn 10 entlang ihrer in Längsrichtung 24 beidseits verlaufenden Faltlinien FW2 50 auf sich selbst gefaltet wird. Die so erhaltene gefaltete Konfiguration kann herstellerseitig vorzugsweise fixiert sein, etwa durch einzelne Fügestellen, insbesondere Klebe- oder Schweiß-, insbesondere Ultraschallschweißstellen, die gleichwohl zum Entfalten der mittels weiterer nachfolgend näher beschriebener Verfahrensschritte, nämlich Schneide-, Vereinzelungs- und Fügeschritte, aus der zweinutzigen Windelseitenteilbahn gebildeten Windelseitenteile durch den Benutzer verhältnismäßig leicht, insbesondere in einem Zug, manuell lösbar sind.

Die auf sich selbst gefaltete zweinutzige Windelseitenteilbahn 10 wird einer Schneidestation S3 zugeführt und dort im zentralen, das Anfügematerial umfassenden Bereich 53 in Längsrichtung 24 in zwei in Querrichtung 25 einnutzigen Windelseitenteilbahnen 13a und 13b getrennt. Die einnutzigen Windelseitenteilbahnen 13a und 13b werden in einer Vereinzelungsstation V1 in Querrichtung 25 in einnutzige Windelseitenteilabschnitte 14a bzw. 14b getrennt. Die Windelseitenteilabschnitte 14a und 14b werden mit einer endlosen Windelhauptteilbahn 15 in einer Fügestation U3 zusammengeführt und bevorzugt mittels Ultraschallschweißen und insbesondere getaktet beidseits an die Windelhauptteilbahn 15 angefügt. Das Vereinzeln der Windelseitenteilabschnitte 14a und 14b und deren Anfügen an die Windelhauptteilbahn 15 kann durch das dem Fachmann an sich bekannte Cut&Place-Verfahren (Slip-Cut) erfolgen, jedoch sind auch alle anderen dem Fachmann an sich bekannten und zu dem Zweck geeigneten Applikationstechniken möglich. Die beidseits Windelseitenteilabschnitte aufweisende Windelhauptteilbahn 15 wird einer zweiten Vereinzelungsstation V2 zugeführt, in der die Windelhauptteilbahn 15 in Querrichtung 25 durchtrennt wird, wobei vereinzelte Inkontinenzwegwerfwindeln 17 gebildet werden. Die Inkontinenzwegwerfwindeln 17 können anschließend weiteren Prozessen wie zusätzlichen Faltungsschritten und/oder der Verpackung zugeführt werden.

Das Aneinanderfügen von Bahnen in den Fügestationen U1 und/oder U2 und/oder U3 wird in diesem Ausführungsbeispiel besonders bevorzugt mittels Ultraschallschweißen durchgeführt, jedoch ist jedes andere dem Fachmann an sich bekannte und zu dem Zweck geeignete Fügeverfahren, insbesondere Thermoschweißen, Nähen, Siegeln oder Kleben, ebenso möglich.

Die Trennung der Materialbahnen in den Schneidestationen S1 und/oder S2 und/oder S3 kann in diesem Beispiel mittels unterschiedlichen und dem Fachmann an sich bekannten Verfahren erfolgen, wie beispielsweise Schneiden, Stanzen, Lasern, Fluid-Schneiden (zum Beispiel Wasserstrahl-Schneiden).

Das Vereinzeln der Windelseitenteilabschnitte 14a und 14b in der Vereinzelungsstation V1 und der Inkontinenzwegwerfwindeln 17 in der Vereinzelungsstation V2 kann mittels unterschiedlichen und an sich dem Fachmann bekannten Verfahren erfolgen, wie beispielsweise Schneiden, Stanzen, Lasern, Fluid-Schneiden (zum Beispiel Wasserstrahl-Schneiden).

Die Figur 2a zeigt in perspektivischer, nicht maßstäblicher, sondern schematischer Darstellung beispielhaft das Bereitstellen der zweiten Materialbahn 7 und der dritten Materialbahn 8 durch Trennen der zweinutzigen Materialbahn 6. Die zweinutzige Materialbahn 6 wird endlos von einer ersten Rolle 20 abgewickelt und entlang ihrer Längsrichtung 24 gefördert. Die Querrichtung 25 der zweinutzigen Materialbahn 6 verläuft orthogonal, also im rechten Winkel 26, zur Längsrichtung 24. An der Schneidestation S2 trennt eine Schneidevorrichtung 21 die zweinutzige Materialbahn 6, die in Querrichtung 25 elastisches Material umfasst (oder daraus besteht), in zwei in Querrichtung 25 einnutzige Materialbahnen, nämlich die zweite Materialbahn 7, die in ihrer Querrichtung 25 ein erstes elastisches Material umfasst oder daraus besteht, und die dritte Materialbahn 8, die in ihrer Querrichtung 25 ein zweites elastisches Material umfasst oder daraus besteht. Das erste elastische Material ist dem des zweiten elastischen Materials vorzugsweise identisch, erstes und zweites elastisches Material bestehen also bevorzugt aus demselben Material. Die zweite Materialbahn 7 und die dritte Materialbahn 8 sind in Querrichtung 25 bevorzugt ungefähr gleich breit ausgebildet, jedoch ist eine andere Variante denkbar, bei der die zweite Materialbahn 7 und die dritte Materialbahn 8 unterschiedlich breit ausgebildet sind.

Die Figur 2b zeigt in perspektivischer, nicht maßstäblicher, sondern schematischer Darstellung beispielhaft das Bereitstellen der ersten Materialbahn 3, der vierten Materialbahn 4 und der fünften Materialbahn 5, wobei die erste Materialbahn 3 im Wesentlichen in Querrichtung 25 undehnbares Anfügematerial umfasst (oder daraus besteht), und wobei die vierte Materialbahn 4 ein erstes in ihrer Querrichtung 25 undehnbares Verschlussträgermaterial umfasst oder daraus besteht, und wobei die fünfte Materialbahn 5 ein zweites in ihrer Querrichtung 25 undehnbares Verschlussträgermaterial umfasst oder daraus besteht, durch Trennen einer in Querrichtung 25 dreinutzigen Materialbahn 2, die im Wesentlichen in Querrichtung 25 undehnbares Material umfasst oder daraus besteht. Die dreinutzige Materialbahn 2 wird endlos von einer zweiten Rolle 22 abgewickelt und entlang ihrer Längsrichtung 24 gefördert. Die Querrichtung 25 der dreinutzigen Materialbahn 2 verläuft orthogonal, also im rechten Winkel 26, zur Längsrichtung 24. An der Schneidestation S1 trennt eine Schneidevorrichtung 23 die dreinutzige Materialbahn 2 in drei in Querrichtung 25 einnutzige Materialbahnen, nämlich die erste Materialbahn 3, die vierte Materialbahn 4 und die fünfte Materialbahn 5. Vorliegend umfassen oder bestehen das erste und das zweite Verschlussträgermaterial und das Anfügematerial aus demselben Material, insbesondere aus demselben Vliesstoffmaterial. Zumindest die vierte Materialbahn 4 und die fünfte Materialbahn 5 sind in Querrichtung 25 bevorzugt ungefähr gleich breit ausgebildet, dabei bevorzugt ungefähr gleich breit oder jeweils breiter als die erste Materialbahn 3. In Figur 2b ist die Schneidevorrichtung 23 durch zwei nebeneinander angeordnete Messer gebildet, so dass die beiden Trennvorgänge ungefähr gleichzeitig erfolgen. Alternativ ist denkbar, dass die Messer entlang der Längsrichtung 24 der dreinutzigen Materialbahn 2 versetzt angeordnet sind derart, dass die beiden Trennvorgänge zeitlich nacheinander erfolgen. Beispielhaft sind in Figur 2b die Materialbahnen so angeordnet, dass die vierte Materialbahn 4 aus einem zentralen Bereich der dreinutzigen Materialbahn 2 gebildet wird und die erste Materialbahn 3 und die fünfte Materialbahn 5 beidseits der Materialbahn 4 aus distalen Bereichen der Materialbahn 2. Denkbar ist jedoch auch jede andere Anordnung der ersten Materialbahn 3, der vierten Materialbahn 4 und der fünften Materialbahn 5 entlang der Querrichtung 25.

Die Figuren 3 a bis c zeigen in beispielhafter Weise eine detaillierte Darstellung der Anordnung und Ausführung der Verfahrensschritte zur Herstellung von Inkontinenzwegwerfwindeln 17.

Figur 3a zeigt nicht maßstäblich, sondern schematisch eine beispielhafte Ausführungsform der Anfügeschritte der mit Bezug zu Figuren 2a und 2b referenzierten ersten Materialbahn 3, zweiten Materialbahn 7, dritten Materialbahn 8, vierten Materialbahn 4 und fünften Materialbahn 5 in Draufsicht.

Die erste Materialbahn 3 weist einen ersten Materialbahnrandbereich 30 und einen zweiten Materialbahnrandbereich 31 auf. Die zweite Materialbahn 7 weist einen dritten Materialbahnrandbereich 32 und einen vierten Materialbahnrandbereich 33 auf. Die dritte Materialbahn 8 weist einen fünften Materialbahnrandbereich 34 und einen sechsten Materialbahnrandbereich 35 auf. Die vierte Materialbahn 4 weist einen siebten Materialbahnrandbereich 38 und einen achten Materialbahnrandbereich 39 auf. Die fünfte Materialbahn 5 weist einen neunten Materialbahnrandbereich 40 und einen zehnten Materialbahnrandbereich 41 auf.

Die erste Materialbahn 3, die eine Breite B1 aufweist, wird mit der zweiten Materialbahn 7, die eine Breite B2 aufweist, derart überlappend geführt, dass der erste Materialbahnrandbereich 30 der ersten Materialbahn 3 mit dem dritten Materialbahnrandbereich 32 der zweiten Materialbahn 7 einen Überlappungsbereich oder Fügebereich mit einer Breite B6 bildet, innerhalb dessen die erste Materialbahn 3 und die zweite Materialbahn 7 in der Fügestation U1 unlösbar miteinander verbunden werden. Die erste Materialbahn 3 wird mit der dritten Materialbahn 8, die eine Breite B3 aufweist, derart überlappend geführt, dass der zweite Materialbahnrandbereich 31 der ersten Materialbahn 3 mit dem fünften Materialbahnrandbereich 34 der dritten Materialbahn 8 einen Überlappungsbereich oder Fügebereich mit einer Breite B7 bildet, innerhalb dessen die erste Materialbahn 3 und die dritte Materialbahn 8 in der Fügestation U1 unlösbar miteinander verbunden werden. Die erste Materialbahn 3, die zweite Materialbahn 7 und die dritte Materialbahn 8 bilden dabei gemeinsam die Kompositbahn 9 mit einer Breite B11 und einer zweiten Symmetrieachse 100. Die Kompositbahn 9 weist weiterhin einen ersten Kompositbahnrandbereich 36 und einen zweiten Kompositbahnrandbereich 37 auf.

Die Kompositbahn 9 wird mit der vierten Materialbahn 4, die eine Breite B4 aufweist, derart überlappend geführt, dass der erste Kompositbahnrandbereich 36 der Kompositbahn 9 mit dem siebten Materialbahnrandbereich 38 der vierten Materialbahn 4 einen Überlappungsbereich oder Fügebereich mit einer Breite B8 bildet, innerhalb dessen die Kompositbahn 9 und die vierte Materialbahn 4 in der Fügestation U2 unlösbar miteinander verbunden werden. Die Kompositbahn 9 wird mit der fünften Materialbahn 5, die eine Breite B5 aufweist, derart überlappend geführt, dass der zweite Kompositbahnrandbereich 37 der Kompositbahn 9 mit dem neunten Materialbahnrandbereich 40 der fünften Materialbahn 5 einen Überlappungsbereich oder Fügebereich mit einer Breite B9 bildet, innerhalb dessen die Kompositbahn 9 und die fünfte Materialbahn 5 in der Fügestation U2 unlösbar miteinander verbunden werden. Die Kompositbahn 9, die vierte Materialbahn 4 und die fünfte Materialbahn 5 bilden dabei gemeinsam die in Querrichtung 25 zweinutzige Windelseitenteilbahn 10 mit einer Breite B10 und einer ersten Symmetrieachse 101.

In dieser beispielhaften Ausführungsform beträgt die Breite B1 100 mm, die Breite B2 90 mm und die Breite B3 90 mm. Die Breite B1 weist hier mit 100 mm einen kleineren Wert auf als die Summe der Breiten B2 und B3, die 180 mm beträgt. Ein Quotient Q1 aus der Breite B1 (Zähler) und der Summe der Breiten B2 und B3 (Nenner) weist in dieser Ausführungsform einen Wert von 0,6 auf [Berechnung: $Q1=(B1/(B2+B3))$, also $Q1= (100\ mm\ /\ (90\ mm + 90\ mm))$].

Die Breite B4 beträgt in dieser Variante 170 mm und die Breite B5 beträgt 170 mm. Ein Quotient Q2 aus der Breite B1 (Zähler) und der Summe der Breiten B4 und B5 (Nenner) weist einen Wert von 0,3 auf [Berechnung: $Q2=(B1/(B4+B5))$, also $Q2=(100\ mm\ /\ (170\ mm + 170\ mm))$].

Die Breite B6 des von der ersten Materialbahn 3 und der zweiten Materialbahn 7 gebildeten Überlappungsbereichs oder Fügebereichs weist einen Wert von 10 mm auf.

Die Breite B7 des von der ersten Materialbahn 3 und der dritten Materialbahn 8 gebildeten Überlappungsbereichs oder Fügebereichs weist einen Wert von 10 mm auf.

Die Breite B8 des von der Kompositbahn 9 und der vierten Materialbahn 4 gebildeten Überlappungsbereichs oder Fügebereichs weist einen Wert von 10 mm auf.

Die Breite B9 des von der Kompositbahn 9 und der fünften Materialbahn 5 gebildeten Überlappungsbereichs oder Fügebereichs weist einen Wert von 10 mm auf.

Die Breite B10 der zweinutzigen Windelseitenteilbahn 10 weist in diesem Ausführungsbeispiel einen Wert von 580 mm auf.

Die Breite B11 der Kompositbahn 9 weist in diesem Ausführungsbeispiel einen Wert von 260 mm auf.

Ein Quotient Q3 aus der Breite 11 (Zähler) und der Breite 10 (Nenner) weist einen Wert von 0,45 auf.

Ein Abstand B2e in Querrichtung 25 der Windelseitenteilbahn 10 zwischen dem vom ersten Materialbahnrandbereich 30 und dem dritten Materialbahnrandbereich 32 gebildeten Überlappungs- oder Fügebereich und dem vom ersten Kompositbahnrandbereich 36 und dem siebten Materialbahnrandbereich 38 gebildeten Überlappungs- oder Fügebereich beträgt in diesem Ausführungsbeispiel 70 mm.

Ein Abstand B3e in Querrichtung 25 der Windelseitenteilbahn 10 zwischen dem vom zweiten Materialbahnrandbereich 31 und dem fünften Materialbahnrandbereich 34 gebildeten Überlappungs- oder Fügebereich und dem vom zweiten Kompositbahnrandbereich 37 und dem neunten Materialbahnrandbereich 40 gebildeten Überlappungs- oder Fügebereich beträgt in diesem Ausführungsbeispiel 70 mm. B2e und B3e bezeichnen mithin die Breiten des jeweiligen effektiven elastischen Bereichs der zweinutzigen Windelseitenteilbahn 10, da die elastischen Eigenschaften der Materialien der zweiten und dritten Materialbahn in den jeweiligen Überlappungs- und Fügebereichen unter Umständen beeinträchtigt sind.

Die zweinutzige Windelseitenteilbahn 10 weist einen ersten Windelseitenteilbahnrand 42 und einen zweiten Windelseitenteilbahnrand 43 auf und wird einer Taper-Station T zugeführt, in der die jeweiligen Verschlusselemente 44 an erste Bereiche des ersten Windelseitenteilbahnrandes 42 und an zweite Bereiche des zweiten Windelseitenteilbahnrandes 43 angefügt werden.

In der in Figur 3a dargestellten bevorzugten Ausführungsform werden die zweite Materialbahn 7 und die dritte Materialbahn 8 gleichzeitig an die erste Materialbahn 3 angefügt, so dass die erste Materialbahn 3 zusammen mit der zweiten Materialbahn 7 und der dritten Materialbahn 8 gemeinsam die Kompositbahn 9 bilden. Nachfolgend werden die vierte Materialbahn 4 und die fünfte Materialbahn 5 wiederum gleichzeitig an die Kompositbahn 9 angefügt. Jede andere Reihenfolge oder Kombination der Anfügeschritte ist ebenso denkbar und von der vorliegenden Erfindung umfasst.

Das Verbinden von Materialbahnrandbereichen in den Fügestationen U1 und/oder U2 wird in diesem Ausführungsbeispiel besonders bevorzugt mittels Ultraschallschweißen durchgeführt, jedoch ist jedes andere dem Fachmann an sich bekannte und zu dem Zweck geeignete Fügeverfahren, insbesondere Thermoschweißen, Nähen, Siegeln oder Kleben, ebenso möglich.

In Figur 3b ist nicht maßstäblich, sondern schematisch und in Draufsicht ein bevorzugtes Ausführungsbeispiel der Faltung der mit Verschlusselementen 44 versehenen und in Querrichtung 25 zweinutzigen Windelseitenteilbahn 10 auf sich selbst und der Trenn- und Vereinzelungsschritte zur Bildung der Windelseitenteilabschnitte 14a und 14b gezeigt. Die zweinutzige Windelseitenteilbahn 10 wird in dieser Variante einer Faltstation F1 zugeführt, in der die zweinutzige Windelseitenteilbahn 10 beidseits um jeweils zwei parallel zur Längsrichtung 24 der zweinutzigen Windelseitenteilbahn 10 verlaufende Faltlinien FW2 50 Z-förmig auf sich selbst gefaltet wird, wobei eine in Querrichtung 25 zweinutzige, mit Verschlusselementen 44 versehene und auf sich selbst gefaltete Windelseitenteilbahn 10 gebildet wird. Der erste Windelseitenteilbahnrand 42 und der zweite Windelseitenteilbahnrand 43 kommen dabei in Querrichtung 25 außerhalb des jeweiligen auf sich selbst gefalteten Bereiches 51, also außerhalb des Bereiches zwischen den beiden Faltlinien FW2 50 zu liegen. Diese Anordnung ist besonders vorteilhaft, da dabei ein in der Querrichtung 25 überstehender distaler Anfassbereich 56 gebildet wird, der das Ergreifen und Entfalten der mittels weiterer nachfolgend näher beschriebener Verfahrensschritte aus der zweinutzigen Windelseitenteilbahn 10 gebildeten Windelseitenteile durch den Benutzer erleichtert.

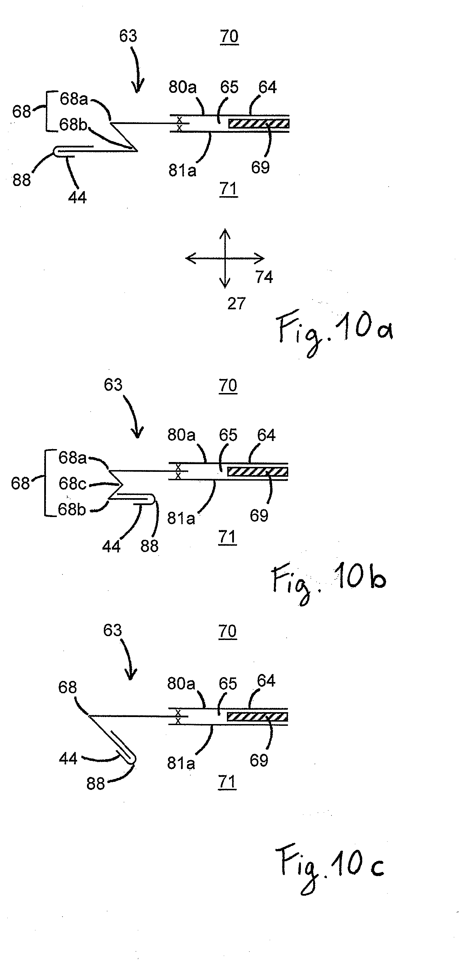

In dieser beispielhaften Ausführung wird die zweinutzige Windelseitenteilbahn 10 beidseits um jeweils zwei Faltlinien FW2 50, Z-förmig, auf sich selbst gefaltet. Als weitere Varianten sind wie in Figuren 10b und 10c (dort aber mit Bezug zur fertigen Inkontinenzwegwerfwindel) illustriert, beidseits jeweils eine einzige Faltlinie FW2, zum Beispiel C-förmig, oder beidseits jeweils mindestens drei Faltlinien FW2, zum Beispiel W-förmig, denkbar, um die die zweinutzige Windelseitenteilbahn 10 auf sich selbst gefaltet wird.

Die in Querrichtung 25 zweinutzige und auf sich selbst gefaltete Windelseitenteilbahn 10 wird weiter zu einer Schneidestation S3 gefördert, in der mittels einer Schneidevorrichtung 52 die zweinutzige Windelseitenteilbahn 10 in Längsrichtung 24 getrennt wird. Die Trennung verläuft dabei im zentralen, das Anfügematerial umfassenden und nicht auf sich selbst gefalteten Bereich 53 der Windelseitenteilbahn 10 derart, dass zwei in Querrichtung 25 einnutzige Windelseitenteilbahnen 13a und 13b gebildet werden. Die in Querrichtung 25 einnutzigen Windelseitenteilbahnen 13a und 13b werden einer Vereinzelungsstation V1 zugeführt, in der die einnutzigen Windelseitenteilbahnen 13a und 13b in Querrichtung 25 durchtrennt werden derart, dass vereinzelte Windelseitenteilabschnitte 14a und 14b gebildet werden. Dabei ist ein das Anfügematerial umfassender Bereich 57 der Windelseitenteilabschnitte 14a bzw. 14b an einem dem jeweiligen Anfassbereich 56 gegenüberliegenden Längsrand 58 des Windelseitenteilabschnittes 14a bzw. 14b angeordnet.

Die noch nicht um die Faltlinien FW2 50 gefaltete zweinutzige Windelseitenteilbahn 10 umfasst in Figur 3b in beispielhaft gezeigter und bevorzugter Weise die Verschlusselemente 44. Jedoch ist auch eine alternative Anordnung der Verfahrensschritte zum Anfügen der Verschlusselemente 44 denkbar, wie beispielsweise ein Anfügen der Verschlusselemente 44 an die in Querrichtung 25 einnutzigen Windelseitenteilbahnen 13a und 13b oder an die bereits vereinzelten Windelseitenteilabschnitte 14a und 14b.

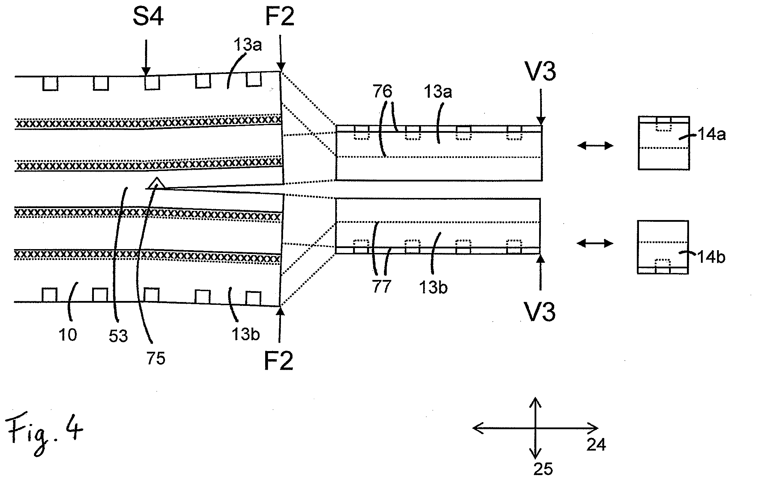

In Figur 3b ist eine bevorzugte Variante dargestellt, bei der die zweinutzige Windelseitenteilbahn 10 zunächst in der Faltstation F1 auf sich selbst gefaltet wird und anschließend in der Schneidestation S3 in Längsrichtung 24 in zwei in Querrichtung 25 einnutzige Windelseitenteilbahnen 13a und 13b getrennt wird. Alternativ ist denkbar, wie in Figur 4 nicht maßstäblich, sondern schematisch und in Draufsicht näher illustriert, dass zunächst die zweinutzige Windelseitenteilbahn 10 in einer Schneidestation S4 mittels einer Schneidevorrichtung 75 im zentralen, das Anfügematerial umfassenden Bereich 53 ungefähr mittig und in Längsrichtung 24 getrennt wird, wobei zwei einnutzige Windelseitenteilbahnen 13a und 13b gebildet werden. Solchen Falls werden die einnutzigen Windelseitenteilbahnen 13a und 13b nachfolgend zu einer Faltstation F2 gefördert und dort entlang jeweils zweier jeweiliger Faltlinien FW3 76 oder Faltlinien FW4 77 jeweils auf sich selbst gefaltet. Die auf sich selbst gefalteten und in Querrichtung 25 einnutzigen Windelseitenteilbahnen 13a und 13b werden einer Vereinzelungsstation V3 zugeführt, in der die einnutzigen Windelseitenteilbahnen 13a und 13b in Querrichtung 25 durchtrennt werden derart, dass vereinzelte Windelseitenteilabschnitte 14a und 14b gebildet werden.

Die Trennung der zweinutzigen Windelseitenteilbahn 10 in der Schneidestation S4 und/oder das Vereinzeln der Windelseitenteilabschnitte 14a und 14b in der Vereinzelungsstation V3 kann mittels unterschiedlichen und dem Fachmann an sich bekannten Verfahren erfolgen, wie beispielsweise Schneiden, Stanzen, Lasern, Fluid-Schneiden (zum Beispiel Wasserstrahl-Schneiden).

Figur 3c zeigt nicht maßstäblich, sondern schematisch und in Draufsicht ein bevorzugtes Ausführungsbeispiel des Anfügens von Windelseitenteilabschnitten 14a und 14b an eine Windelhauptteilbahn 15. Die Windelhauptteilbahn 15 wird zu einer Fügestation U3 gefördert. In der Fügestation U3 werden die je ein Verschlusselement 44 umfassenden Windelseitenteilabschnitte 14a und 14b der Windelhauptteilbahn 15 paarweise zugeführt und zwar jeweils bevorzugt in der Längsrichtung 24 getaktet, bevorzugt beidseitig und bevorzugt in der Querrichtung 25 ungefähr einander gegenüberliegend. Die Windelseitenteilabschnitte 14a und 14b werden dabei so angeordnet, dass sie jeweils zumindest mit einem Teil des jeweiligen das Anfügematerial umfassenden Bereiches 57 an dem dem Anfassbereich 56 gegenüberliegenden Längsrand 58 des jeweiligen Windelseitenteilabschnittes 14a bzw. 14b einen Überlappungs- oder Fügebereich mit jeweils einem ersten Bereich 60 oder einem zweiten Bereich 61 der Windelhauptteilbahn 15 bilden, innerhalb dessen der jeweilige Windelseitenteilabschnitt 14a bzw. 14b und die Windelhauptteilbahn 15 in der Fügestation U3 unlösbar miteinander verbunden werden, wobei der Windelseitenteilabschnitt 14a das erste elastische Windelseitenteil 62 und der Windelseitenteilabschnitt 14b das zweite elastische Windelseitenteil 63 bildet.

Das Verbinden der Windelseitenteilabschnitte 14a und 14b mit der Windelhauptteilbahn 15 in der Fügestation U3 wird in diesem Ausführungsbeispiel besonders bevorzugt mittels Ultraschallschweißen durchgeführt, jedoch ist jedes andere dem Fachmann an sich bekannte und zu dem Zweck geeignete Fügeverfahren, insbesondere Thermoschweißen, Nähen, Siegeln oder Kleben, ebenso möglich.

Die Windelhauptteilbahn 15 wird einer Vereinzelungsstation V2 zugeführt, in der die Inkontinenzwegwerfwindeln 17 vereinzelt werden, indem die Windelhauptteilbahn 15 in Querrichtung 25 getrennt wird.

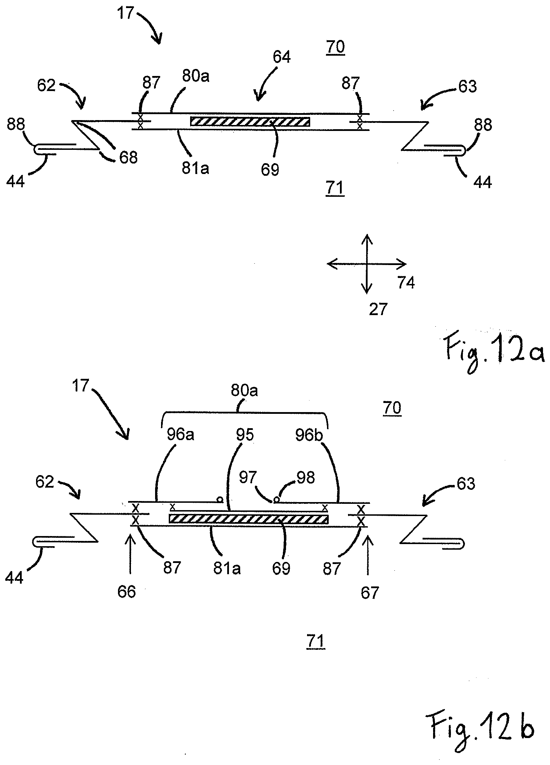

In der dargestellten Ausführungsform besteht die Windelhauptteilbahn 15 aus zwei Flachmaterialbahnen, nämlich aus einer zumindest bereichsweise flüssigkeitsdurchlässigen Deckschichtbahn 80 (Topsheet) und einer zumindest bereichsweise flüssigkeitsundurchlässigen Rückschichtbahn 81 (Backsheet), zwischen die, in Längsrichtung getaktet, flüssigkeitsabsorbierende Saugkörper 69 eingebracht sind. Die Windelseitenteilabschnitte 14a und 14b werden mit ihren das Anfügematerial umfassenden Bereichen 57 zwischen Topsheetbahn 80 und Backsheetbahn 81 platziert, wie mit Bezug zu Figur 12a näher beschrieben. Alternativ ist es möglich (siehe Figur 12b), dass die Topsheetbahn 80 beidseits in Querrichtung 25 der Windelhauptteilbahn 15 (bzw. in Windelquerrichtung 74, vgl. Figur 12b) flüssigkeitsundurchlässiges Material (Cuffvlies) mit der Funktion einer seitlichen Auslaufsperre aufweist, wobei solchen Falls das erste elastische Windelseitenteil 62 und das zweite elastische Windelseitenteil 63 zwischen der Backsheetbahn 81 und dem Cuffvlies platziert sind. Jede andere Platzierung des ersten elastischen Windelseitenteils und des zweiten elastischen Windelseitenteils der Inkontinenzwegwerfwindel in einer Dickenrichtung ist jedoch ebenso denkbar.

Nach einer bevorzugten Variante (nicht dargestellt in Figur 3c) werden die Windelseitenteilabschnitte 14a, 14b nach dem Anfügen der Windelseitenteilabschnitte 14a, 14b an die Windelhauptteilbahn 15, aber vor dem Vereinzeln der Inkontinenzwegwerfwindel 17, auf die Windelhauptteilbahn 15 gefaltet.

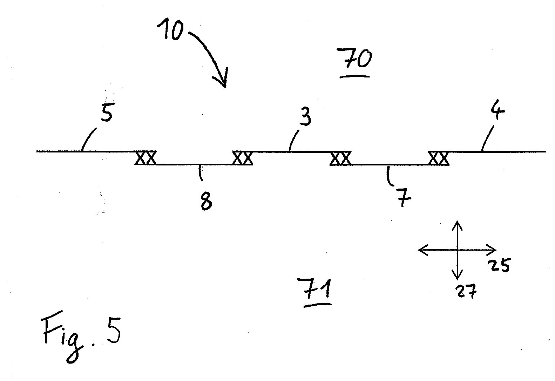

In Figur 5 ist nicht maßstäblich, sondern schematisch und im Querschnitt ein bevorzugtes Ausführungsbeispiel einer in Querrichtung 25 zweinutzigen Windelseitenteilbahn 10 dargestellt. Die erste Materialbahn 3, die zweite Materialbahn 7, die dritte Materialbahn 8, die vierte Materialbahn 4 und die fünfte Materialbahn 5 sind in Querrichtung 25 nebeneinander angeordnet und zwar derart, dass die jeweiligen direkt benachbarten Materialbahnen mit ihren jeweiligen in Richtung der nächstgelegenen Materialbahn weisenden Bahnrandbereichen jeweils einen Überlappungsbereich oder Fügebereich bilden, innerhalb dessen die jeweiligen Bahnen unlösbar miteinander verbunden sind. Bevorzugt ist wie in Figur 5 dargestellt in den jeweiligen Füge- oder Überlappungsbereichen eine im Tragezustand der Inkontinenzwegwerfwindel körperzugewandte Oberseite 70 der zweiten Materialbahn 7 und der dritten Materialbahn 8 mit einer körperabgewandten Oberseite 71 der ersten Materialbahn 3, der vierten Materialbahn 4 und der fünften Materialbahn 5 verbunden. Jede andere Anordnung der Bahnen in der Dickenrichtung 27 ist jedoch ebenso denkbar und als mögliche Ausführungsform umfasst.

Die Figur 6 zeigt, nicht maßstäblich, sondern schematisch eine insgesamt mit dem Bezugszeichen 17 bezeichnete Inkontinenzwegwerfwindel in der sogenannten T-Form. Die Inkontinenzwegwerfwindel 17 umfasst einen insgesamt mit dem Bezugszeichen 65 bezeichneten Hauptteil mit einem Körperflüssigkeiten absorbierenden Saugkörper 69. Der Saugkörper 69 umfasst vorzugsweise Zellulosefasern und superabsorbierende Polymerpartikel (SAP). Der Saugkörper 69 ist zwischen zwei Flachmaterialien, und zwar einer flüssigkeitsdurchlässigen Deckschicht 80a (Topsheet) und einer flüssigkeitsundurchlässigen Rückschicht 81a (Backsheet) des Windelhauptteils 65 angeordnet.

Bei der Inkontinenzwegwerfwindel 17 ist eine Windellängsrichtung 73 und eine Windelquerrichtung 74 unterscheidbar, wobei letztere im angelegten Zustand der Inkontinenzwegwerfwindel der Hüftumfangsrichtung des Benutzers entspricht. Der Hauptteil 65 umfasst einen Vorderbereich 82 mit vorderen seitlichen Längsrändern 83, einen Rückenbereich 64 mit einem ersten hinteren seitlichen Längsrand 66 und einem zweiten hinteren seitlichen Längsrand 67 und einen dazwischen angeordneten Schrittbereich 84. An einem jeweiligen Längsrand 85 des Schrittbereichs 84 angrenzend weist der Hauptteil 65 je einen elastifizierten Abschnitt 86, mithin einen elastifizierten Beinöffnungsabschnitt auf. Diese elastifizierten Beinöffnungsabschnitte 86 sind gebildet durch zwischen Topsheet 80a und Backsheet 81a verlaufende und in vorgespanntem Zustand an Topsheet 80a und/oder Backsheet 81a fixierte elastische Fäden, die bogenförmig gekrümmt, mithin zumindest abschnittsweise in Windellängsrichtung 73 orientiert sind.

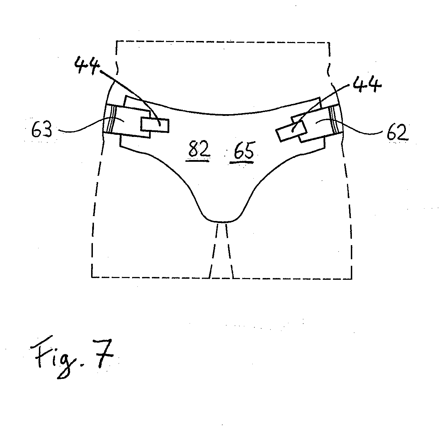

Bei der T-förmigen Inkontinenzwegwerfwindel 17 ist im Rückenbereich 64 des Hauptteils 65 in Windelquerrichtung 74 ein seitlich über den ersten hinteren seitlichen Längsrand 66 hinaus erstrecktes und durch den Windelseitenteilabschnitt 14a (siehe auch Figuren 3b und 3c) gebildetes erstes elastisches Windelseitenteil 62 und ein seitlich über den zweiten hinteren seitlichen Längsrand 67 hinaus erstrecktes und durch den Windelseitenteilabschnitt 14b (siehe auch Figuren 3b und 3c) gebildetes zweites elastisches Windelseitenteil 63 vorgesehen, die im Bereich der hinteren seitlichen Längsränder 66 bzw. 67 in einem Überlappungsbereich 87 unlösbar an den Rückenbereich 64 des Hauptteils 65 angefügt sind. Im Vorderbereich 82 sind hingegen keine Windelseitenteile vorgesehen. Das erste elastische Windelseitenteil 62 und das zweite elastische Windelseitenteil 63 haben im Bereich ihres in Windelquerrichtung 74 freien Endes 88 jeweils wenigstens ein Verschlusselement 44. Das Verschlusselement 44 ist in Form einer vorzugsweise rechteckigen Lasche ausgebildet und herstellerseitig auf sich selbst eingefaltet. In der Gebrauchssituation lässt sich das Verschlusselement 44 öffnen, das heißt wieder auffalten, um die Inkontinenzwegwerfwindel 17 an einen Benutzer anzulegen, wobei das erste elastische Windelseitenteil 62 und das zweite elastische Windelseitenteil 63 in Überlappung mit dem Vorderbereich 82 des Hauptteils 65 gebracht werden und die Verschlusselemente 44 lösbar haftend auf der vom Benutzer abgewandten Seite des Vorderbereichs 82 des Hauptteils 65 festgelegt werden (schematisch dargestellt in Figur 7).

Das erste elastische Windelseitenteil 62 und das zweite elastische Windelseitenteil 63 haben in der Windellängsrichtung 73 einen Abstand D von vorzugsweise 5 - 50 mm von einem hinteren Querrand 79 der Inkontinenzwegwerfwindel 17.

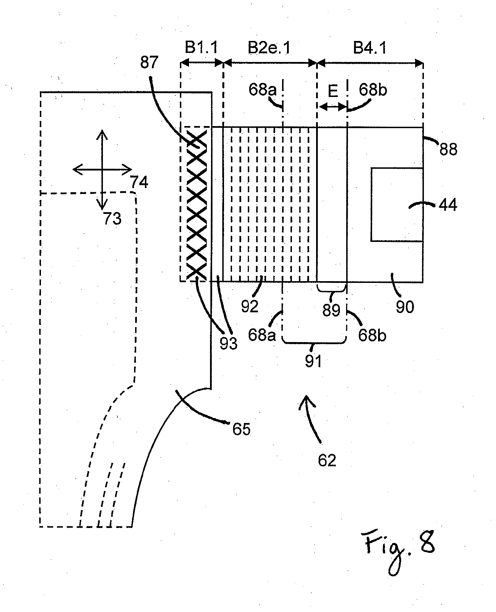

Figur 8 zeigt einen Ausschnitt der Inkontinenzwegwerfwindel 17 nach Figur 6 in vergrößerter Darstellung, und zwar im Bereich eines ersten elastischen Windelseitenteils 62 in eben ausgebreitetem, jedoch nicht gedehntem Zustand mit zwei Faltlinien FW1 68a und 68b.

Wenn im Folgenden das erste elastische Windelseitenteil 62 beschrieben wird, so wird das Verständnis unterstellt, dass das zweite elastische Windelseitenteil 63 lediglich seitenverkehrt vorzugsweise aber im Übrigen identisch konstruiert und bemessen ist. Das Gleiche gilt umgekehrt, wenn weiter unten das zweite elastische Windelseitenteil 63 beschrieben wird.

Im vorliegenden Fall entsprechen die Faltlinien FW1 68a, 68b den Faltlinien FW2 50 der zweinutzigen Windelseitenteilbahn 10 (vgl. Figur 3b). Der Bildung der Windelseitenteilabschnitte wie zuvor mit Bezug zu Figuren 1-4 beschrieben entsprechend, umfasst das erste elastische Windelseitenteil 62 in Windelquerrichtung 74 drei aneinander direkt anschließende Bereiche, nämlich einen im Wesentlichen undehnbaren distalen Bereich 90, einen im Wesentlichen undehnbaren proximalen Bereich 93 und einen dazwischenliegenden elastischen Bereich 92.

Der distale Bereich 90 des ersten elastischen Windelseitenteils 62 weist die Erstreckung B4.1 in Windelquerrichtung 74 auf (wobei B4.1 im Wesentlichen der Breite B4, siehe Figur 3a, entspricht), umfasst im Wesentlichen undehnbares Verschlussträgermaterial und bildet das freie Ende 88 des ersten elastischen Windelseitenteils 62. Durch diesen distalen Bereich 90 verläuft in Windellängsrichtung 73 die dem Verschlusselement 44 oder dem freien Ende 88 des ersten elastischen Windelseitenteils 62 am nächsten gelegene Faltlinie FW1 68b. Der im Wesentlichen undehnbare proximale Bereich 93 weist eine ungefähr der halben Breite B1 der ersten Materialbahn 3 (siehe Figur 3a) entsprechende Erstreckung B1.1 in Windelquerrichtung 74 auf und umfasst das im Wesentlichen undehnbare Anfügematerial. Der proximale Bereich 93 überlappt mit dem Windelhauptteil 65 und ist im Überlappungs- oder Fügebereich 87 wie in Figur 3c näher illustriert unlösbar mit dem Windelhauptteil 65 verbunden.

Der zwischen dem distalen Bereich 90 und dem proximalen Bereich 93 liegende und zumindest in Windelquerrichtung 74 elastische Bereich 92 weist eine Erstreckung B2e.1 in Windelquerrichtung 74 auf (welche der Erstreckung B2e, Figur 3a, entspricht). Durch den elastischen Bereich 92 verläuft in Windellängsrichtung 73 die dem Windelhauptteil 65 am nächsten gelegene Faltlinie FW1 68a.

In diesem Ausführungsbeispiel beträgt eine Erstreckung E eines undehnbaren Teilbereiches 89 des in Windelquerrichtung 74 zwischen den beiden Faltlinien FW1 68a und 68b sich erstreckenden Teilbereichs 91 80 mm.

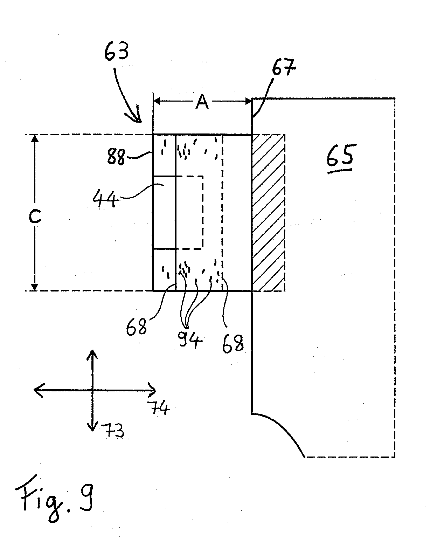

Figur 9 zeigt einen Ausschnitt der Inkontinenzwegwerfwindel 17 nach Figur 6 in vergrößerter Darstellung, und zwar im Bereich eines zweiten elastischen Windelseitenteils 63 mit Verschlusselement 44 in gefalteter Konfiguration mit zwei Faltlinien FW1 68, in Z-Konfiguration.

Eine Erstreckung C des Windelseitenteils 63 in der Windellängsrichtung 73 beträgt im dargestellten Fall 140 mm. Eine Erstreckung A des in Z-Konfiguration gefalteten Windelseitenteils 63 gemessen vom freien Ende 88 des zweiten elastischen Windelseitenteils 63 bis zum zweiten hinteren seitlichen Längsrand 67 des Hauptteils 65 in der Windelquerrichtung 74 beträgt im dargestellten Fall 80 mm. Das Verhältnis der Erstreckungen A/C zueinander beträgt 0,6 [Berechnung: A/C=(80 mm / 140 mm)].

In dieser Darstellung ist die gefaltete Konfiguration des zweiten elastischen Windelseitenteils 63 durch Fügestellen 94 in der Z-Konfiguration lösbar fixiert.

Figur 10a zeigt eine Schnittansicht in der Schnittebene III-III aus Figur 6. Die Schnittebene verläuft in diesem Ausführungsbeispiel in Windelquerrichtung 74 im Rückenbereich 64 einer Inkontinenzwegwerfwindel 17. In dieser nicht maßstäblich, sondern schematisch dargestellten beispielhaften Ausführungsform ist zwischen der Deckschicht 80a (Topsheet), die die körperzugewandte Oberseite 70 des Windelhauptteils 65 bildet, und der Rückschicht 81a (Backsheet), die die körperabgewandte Oberseite 71 des Windelhauptteils 65 bildet, und die gemeinsam einen Saugkörper 69 umschließen, im Rückenbereich 64 der Inkontinenzwegwerfwindel 17 das zweite elastische Windelseitenteil 63 platziert. Jede andere Platzierung des zweiten elastischen Windelseitenteils 63 in der Dickenrichtung 27 der Inkontinenzwegwerfwindel 17 ist jedoch ebenso denkbar und als mögliche Ausführungsform umfasst.

Das zweite elastische Windelseitenteil 63 ist in dieser Ausführung um zwei Faltlinien FW1 68 auf sich selbst gefaltet, wobei eine sogenannte Z-Konfiguration gebildet wird, dabei ist das zweite elastische Windelseitenteil 63 an der dem Hauptteil 65 im plan ausgebreiteten Zustand, also im nicht gefalteten Zustand, am nächsten liegenden Faltlinie FW1 68a in Richtung der körperabgewandten Oberseite 71 und an der dem jeweiligen Verschlusselement 44 oder dem freien Ende 88 des zweiten elastischen Windelseitenteils 63 im plan ausgebreiteten Zustand, also im nicht gefalteten Zustand, am nächsten liegenden Faltlinie FW1 68b in Richtung der körperzugewandten Oberseite 70 der Inkontinenzwegwerfwindel 17 gefaltet. Diese Konfiguration hat sich als besonders vorteilhaft beim Entfalten der Windelseitenteile durch den Anwender erwiesen. Jedoch ist auch die umgekehrte Orientierung der Faltungen um die Faltlinien 68a und 68b möglich.

In Figuren 10b und 10c sind alternative Ausführungsformen der Faltung des zweiten elastischen Windelseitenteils 63 in jeweils einer nicht maßstäblichen, sondern schematischen Schnittansicht dargestellt.

Figur 10b zeigt ein um drei Faltlinien FW1 68 W-förmig auf sich selbst gefaltetes zweites elastisches Windelseitenteil 63, wobei das zweite elastische Windelseitenteil 63 an der dem Hauptteil 65 im plan ausgebreiteten Zustand, also im nicht gefalteten Zustand, am nächsten liegenden Faltlinie FW1 68a und an der dem Verschlussteil 44 oder dem freien Ende 88 des zweiten elastischen Windelseitenteils 63 im plan ausgebreiteten Zustand, also im nicht gefalteten Zustand, am nächsten liegenden Faltlinie FW1 68b jeweils in Richtung der körperabgewandten Oberseite 71 der Inkontinenzwegwerfwindel 17 gefaltet ist, und wobei das zweite elastische Windelseitenteil 63 an der mittleren Faltlinie FW1 68c in Richtung der körperzugewandten Oberseite 70 der Inkontinenzwegwerfwindel 17 gefaltet ist. Es ist auch die umgekehrte Orientierung der Faltungen um die Faltlinien 68a, 68b und 68c denkbar.

Figur 10c zeigt ein um eine einzige Faltlinie FW1 68 C-förmig auf sich selbst gefaltetes zweites elastisches Windelseitenteil 63, wobei das zweite elastische Windelseitenteil 63 an der Faltlinie FW1 68 in Richtung der körperabgewandten Oberseite 71 der Inkontinenzwegwerfwindel 17 gefaltet ist. Es ist auch die umgekehrte Orientierung der Faltung um die Faltlinie FW1 68 denkbar.

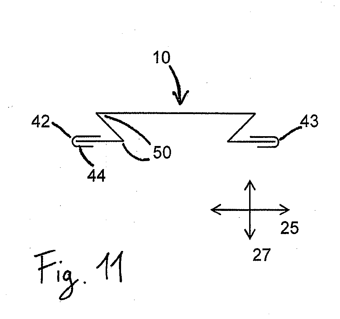

In Figur 11 ist nicht maßstäblich, sondern schematisch eine Schnittansicht in der Schnittebene I-I aus Figur 3b gezeigt. Die Schnittebene verläuft in Querrichtung 25 durch die zweinutzige Windelseitenteilbahn 10, die beidseits, also an ihrem ersten 42 und zweiten Windelseitenteilbahnrand 43 mit jeweils einem Verschlusselement 44 versehen und beidseits jeweils um zwei parallel zur Längsrichtung 24 verlaufende Faltlinien FW2 50 Z-förmig auf sich selbst gefaltet ist. Die zweinutzige Windelseitenteilbahn 10 ist in Figur 11 vereinfacht dargestellt, nämlich ohne Kennzeichnung der Überlappungs- oder Fügebereiche der die Windelseitenteilbahn 10 bildenden Bahnen.

Figur 12a zeigt nicht maßstäblich, sondern schematisch eine Schnittansicht in der Schnittebene II-II aus Figur 3c. Die Schnittebene verläuft in Windelquerrichtung 74 durch den Rückenbereich 64 der Inkontinenzwegwerfwindel 17. Der Saugkörper 69 ist zwischen einem auf der körperabgewandten Seite 71 der Inkontinenzwegwerfwindel 17 angeordneten Backsheet 81a und einem auf der körperzugewandten Seite 70 der Inkontinenzwegwerfwindel 17 angeordneten Topsheet 80a platziert. Das erste elastische Windelseitenteil 62 und das zweite elastische Windelseitenteil 63 sind an ihrem jeweiligen freien Ende 88 mit jeweils einem Verschlusselement 44 versehen und jeweils um zwei Faltlinien FW1 68 Z-förmig auf sich selbst gefaltet. Das erste 62 und das zweite Windelseitenteil 63 ist jeweils zwischen dem auf der körperabgewandten Seite 71 der Inkontinenzwegwerfwindel 17 angeordneten Backsheet 81a und dem auf der körperzugewandten Seite 70 der Inkontinenzwegwerfwindel 17 angeordneten Topsheet 80a angeordnet und in dem jeweiligen Überlappungsbereich 87 mit dem Topsheet und/oder mit dem Backsheet unlösbar verbunden. Das Topsheet 80a ist in diesem bevorzugten Ausführungsbeispiel durchgängig aus einem einzigen Flachmaterial gebildet.

In Figur 12b ist eine alternative Ausführungsform einer Inkontinenzwegwerfwindel 17 als nicht maßstäbliche, sondern schematische Schnittansicht dargestellt. Die Schnittebene verläuft in Windelquerrichtung 74 durch die Inkontinenzwegwerfwindel 17. In dieser Variante ist das auf der körperzugewandten Seite der Inkontinenzwegwerfwindel 17 angeordnete Topsheet 80a aus drei Flachmaterialbahnen 95, 96a und 96b ausgebildet. Die drei Flachmaterialbahnen 95, 96a und 96b sind in Windelquerrichtung 74 zueinander versetzt angeordnet und zwar derart, dass das in Windelquerrichtung 74 zentral platziertes vorzugsweise flüssigkeitsdurchlässigen Flachmaterials 95 zwischen einem vorzugsweise flüssigkeitsundurchlässigen ersten Cuffmaterial 96a und einem vorzugsweise flüssigkeitsundurchlässigen zweiten Cuffmaterial 96b angeordnet ist, wobei das erste 96a und das zweite Cuffmaterial 96b jeweils zumindest bereichsweise mit dem zentralen Flachmaterial 95 überlappt, und wobei das erste 96a und das zweite Cuffmaterial 96b in jeweiligen Füge- oder Überlappungsbereichen mit dem zentralen Flachmaterial 95 insbesondere unlösbar verbunden sind. In Dickenrichtung 27 sind die drei das Topsheet 80a bildenden Flachmaterialbahnen 95, 96a und 96b derart angeordnet, dass in den jeweiligen Füge- oder Überlappungsbereichen eine im Tragezustand der Inkontinenzwegwerfwindel 17 körperzugewandte Oberseite 70 des zentralen flüssigkeitsdurchlässigen Flachmaterials 95 mit einer körperabgewandten Oberseite 71 des ersten 96a und des zweiten Cuffmaterials 96b verbunden ist. Das erste 96a und zweite Cuffmaterial 96b erstreckt sich jeweils über den Füge- oder Überlappungsbereich hinaus in Richtung auf das jeweils andere Cuffmaterial und zwar derart, dass die überstehenden freien Bereiche 97 des Cuffmaterials als seitliche Auslaufsperre der Inkontinenzwegwerfwindel 17 fungieren können. Das erste 96a und zweite Cuffmaterial 96b ist in diesem Ausführungsbeispiel im überstehenden freien Bereich 97 insbesondere mit jeweils einem oder mehreren Elastifizierungsmitteln 98 ausgestattet, die ein Emporerheben des ersten 96a und zweiten Cuffmaterials 96b im Tragezustand und deren Funktion als Auslaufsperre unterstützen.

In diesem in Figur 12b dargestellten Ausführungsbeispiel ist das flüssigkeitsdurchlässige Flachmaterial 95 des Topsheets 80a in der Windelquerrichtung 74 in etwa gleich lang erstreckt wie der Saugkörper 69 ausgebildet, insbesondere kürzer als das Backsheet 81a, wobei sich weiter insbesondere das erste Cuffmaterial 96a bis zum ersten hinteren seitlichen Längsrand 66 und das zweite Cuffmaterial 96b bis zum zweiten hinteren seitlichen Längsrand 67 der Inkontinenzwegwerfwindel 17 erstreckt. Dabei sind das erste elastische Windelseitenteil 62 und das zweite elastische Windelseitenteil 63 in diesem Beispiel in Dickenrichtung 27 bevorzugt zwischen dem Backsheet 81a und dem jeweiligen ersten 96a oder zweiten Cuffmaterial 96b platziert und insbesondere mit diesen im jeweiligen Überlappungsbereich 87 unlösbar verbunden.

**Patentansprüche**

1. Verfahren zur Herstellung von Inkontinenzwegwerfwindeln (17), wobei eine jeweilige Inkontinenzwegwerfwindel (17) eine Windellängsrichtung (73) und eine Windelquerrichtung (74) aufweist, und wobei die Inkontinenzwegwerfwindel (17) einen mit einem Saugkörper (69) versehenen Hauptteil (65) aufweist, wobei der Hauptteil (65) einen Rückenbereich (64) mit einem ersten hinteren seitlichen Längsrand (66) und mit einem zweiten hinteren seitlichen Längsrand (67) aufweist, und wobei an den ersten hinteren seitlichen Längsrand (66) ein sich in der Windelquerrichtung (74) über den ersten hinteren seitlichen Längsrand (66) des Hauptteils (65) hinaus erstreckendes erstes elastisches Windelseitenteil (62) angefügt ist, und wobei an den zweiten hinteren seitlichen Längsrand (67) ein sich in der Windelquerrichtung (74) über den zweiten hinteren seitlichen Längsrand (67) des Hauptteils (65) hinaus erstreckendes zweites elastisches Windelseitenteil (63) angefügt ist, umfassend die folgenden Schritte

   - Fördern einer ersten Materialbahn (3), einer zweiten Materialbahn (7), einer dritten Materialbahn (8), einer vierten Materialbahn (4) und einer fünften Materialbahn (5), wobei die erste Materialbahn (3) eine Breite B1 und einen ersten Materialbahnrandbereich (30) und einen zweiten Materialbahnrandbereich (31) aufweist und ein in einer Querrichtung (25) der ersten Materialbahn (3) im Wesentlichen undehnbares Anfügematerial umfasst oder daraus besteht, wobei die zweite Materialbahn (7) eine Breite B2 und einen dritten Materialbahnrandbereich (32) und einen vierten Materialbahnrandbereich (33) aufweist und ein erstes in einer Querrichtung (25) der zweiten Materialbahn (7) zumindest bereichsweise elastisches Material umfasst oder daraus besteht, wobei die dritte Materialbahn (8) eine Breite B3 und einen fünften Materialbahnrandbereich (34) und einen sechsten Materialbahnrandbereich (35) aufweist und ein zweites in einer Querrichtung (25) der dritten Materialbahn (8) zumindest bereichsweise elastisches Material umfasst oder daraus besteht, wobei die vierte Materialbahn (4) eine Breite B4 und einen siebten Materialbahnrandbereich (38) und einen achten Materialbahnrandbereich (39) aufweist und ein erstes in einer Querrichtung (25) der vierten Materialbahn (4) im Wesentlichen undehnbares Verschlussträgermaterial umfasst oder daraus besteht, und wobei die fünfte Materialbahn (5) eine Breite B5 und einen neunten Materialbahnrandbereich (40) und einen zehnten Materialbahnrandbereich (41) aufweist und ein zweites in einer Querrichtung (25) der fünften Materialbahn (5) im Wesentlichen undehnbares Verschlussträgermaterial umfasst oder daraus besteht, und wobei die Materialbahnrandbereiche der ersten (3), zweiten (7), dritten (8), vierten (4) und fünften Materialbahn (5) derart aneinandergefügt werden, dass die erste (3), zweite (7), dritte (8), vierte (4) und fünfte Materialbahn (5) eine in ihrer jeweiligen Querrichtung (25) zweinutzige Windelseitenteilbahn (10) bilden, derart, dass ein zentraler Bereich (53) der zweinutzigen Windelseitenteilbahn (10) durch die erste Materialbahn (3) gebildet wird, dass ein erster äußerer Bereich der zweinutzigen Windelseitenteilbahn (10) durch die vierte Materialbahn (4) gebildet wird, dass ein zweiter äußerer Bereich der zweinutzigen Windelseitenteilbahn (10) durch die fünfte Materialbahn (5) gebildet wird, und dass ein in der Querrichtung (25) der zweinutzigen Windelseitenteilbahn (10) zwischen dem ersten äußeren Bereich und dem zentralen Bereich (53) angeordneter erster elastischer Bereich durch die zweite Materialbahn (7) gebildet wird, und dass ein in der Querrichtung (25) der zweinutzigen Windelseitenteilbahn (10) zwischen dem zweiten äußeren Bereich und dem zentralen Bereich (53) angeordneter zweiter elastischer Bereich durch die dritte Materialbahn (8) gebildet wird,
- Trennen der zweinutzigen Windelseitenteilbahn (10) in einer Längsrichtung (24) der zweinutzigen Windelseitenteilbahn (10) und durch den zentralen Bereich (53) der zweinutzigen Windelseitenteilbahn (10) hindurch, zur Bildung einer ersten (13a) und zweiten jeweils in ihrer jeweiligen Querrichtung (25) einnutzigen Windelseitenteilbahn (13b),
- Vereinzeln von Windelseitenteilabschnitten (14a, 14b) durch Trennen der ersten (13a) und der zweiten einnutzigen Windelseitenteilbahn (13b) in der jeweiligen Querrichtung (25) der ersten (13a) und zweiten einnutzigen Windelseitenteilbahn (13b),
- Zuführen einer Windelhauptteilbahn (15) mit einem ersten Windelhauptteilbahnrand und einem zweiten Windelhauptteilbahnrand,
- Anfügen der Windelseitenteilabschnitte (14a, 14b) an ersten Bereichen (60) des ersten Windelhauptteilbahnrands und an zweiten Bereichen (61) des zweiten Windelhauptteilbahnrands,
- Vereinzeln der Inkontinenzwegwerfwindeln (17) durch Trennen der Windelhauptteilbahn (15) in einer Querrichtung (25) der Windelhauptteilbahn (15).

2. Verfahren nach Anspruch 1, wobei

   - der dritte Materialbahnrandbereich (32) der zweiten Materialbahn (7) an den ersten Materialbahnrandbereich (30) der ersten Materialbahn (3) angefügt wird, und wobei der fünfte Materialbahnrandbereich (34) der dritten Materialbahn (8) an den zweiten Materialbahnrandbereich (31) der ersten Materialbahn (3) angefügt wird, derart dass die erste (3), zweite (7) und dritte Materialbahn (8) gemeinsam eine Kompositbahn (9) bilden, und wobei die Kompositbahn (9) einen ersten Kompositbahnrandbereich (36) und einen zweiten Kompositbahnrandbereich (37) aufweist, und wobei
   - der siebte Materialbahnrandbereich (38) (der vierten Materialbahn (4)) an den ersten Kompositbahnrandbereich (36) der Kompositbahn (9) angefügt wird, und wobei der neunte Materialbahnrandbereich (40) der fünften Materialbahn (5) an den zweiten Kompositbahnrandbereich (37) der Kompositbahn (9) angefügt wird, derart dass die Kompositbahn (9) gemeinsam mit der vierten (4) und fünften Materialbahn (5) die in ihrer Querrichtung (25) zweinutzige Windelseitenteilbahn (10) bilden.

3. Verfahren nach Anspruch 1 oder 2, wobei das erste (62) und das zweite elastische Windelseitenteil (63) jeweils mindestens ein Verschlusselement (44) aufweisen.

4. Verfahren nach einem oder mehreren der vorstehenden Ansprüche, wobei die zweite (7) und dritte Materialbahn (8) durch Trennen einer ersten in ihrer Querrichtung (25) zweinutzigen Materialbahn (6) bereitgestellt werden.

5. Verfahren nach einem oder mehreren der vorstehenden Ansprüche, wobei die vierte (4) und fünfte Materialbahn (5) durch Trennen einer zweiten in ihrer Querrichtung zweinutzigen Materialbahn bereitgestellt werden.

6. Verfahren nach einem oder mehreren der vorstehenden Ansprüche 1 bis 4, wobei die erste (3), vierte (4) und fünfte Materialbahn (5) durch Trennen einer in ihrer Querrichtung (25) dreinutzigen Materialbahn (2) bereitgestellt werden.

7. Verfahren nach einem oder mehreren der vorstehenden Ansprüche, wobei die Windelseitenteilabschnitte (14a, 14b) in ihrer Längsrichtung (24) einnutzig ausgebildet sind.

8. Verfahren nach einem oder mehreren der Ansprüche 1 bis 6, wobei die Windelseitenteilabschnitte in ihrer Längsrichtung zweinutzig ausgebildet sind, und wobei das Trennen der Windelhauptteilbahn (15) durch die Windelseitenteilabschnitte hindurch geführt wird derart, dass ein erster Teilabschnitt eines jeweiligen Windelseitenteilabschnittes ein hinteres Windelseitenteil einer ersten Inkontinenzwegwerfwindel (17) bildet und ein zweiter Teilabschnitt des jeweiligen Windelseitenteilabschnittes ein hinteres Windelseitenteil einer in einer Windellängsrichtung (73) unmittelbar angrenzenden zweiten Inkontinenzwegwerfwindel (17) bildet.

9. Verfahren nach einem oder mehreren der vorstehenden Ansprüche 3 bis 8, wobei die jeweiligen Verschlusselemente (44) an erste Bereiche eines ersten Windelseitenteilbahnrandes (42) und an zweite Bereiche eines zweiten Windelseitenteilbahnrandes (43) angefügt werden.

10. Verfahren nach einem oder mehreren der vorstehenden Ansprüche, **dadurch gekennzeichnet, dass** das erste (62) und das zweite elastische Windelseitenteil (63) an jeweils mindestens einer (68), insbesondere jeweils mindestens zwei (68a, 68b), weiter insbesondere jeweils mindestens drei (68a, 68b, 68c) vorzugsweise parallel zur Windellängsrichtung (73) der Inkontinenzwegwerfwindel (17) sich erstreckenden Faltlinien FW1 (68, 68a, 68b, 68c) (auf sich selbst) gefaltet und insbesondere in dieser Konfiguration lösbar fixiert ist.

11. Verfahren nach einem oder mehreren der vorstehenden Ansprüche, wobei die zweinutzige Windelseitenteilbahn (10) beidseitig um jeweils mindestens eine, insbesondere um jeweils mindestens zwei, weiter insbesondere um jeweils mindestens drei zur Längsrichtung (24) der zweinutzigen Windelseitenteilbahn (10) parallelen Faltlinien FW2 (50) auf sich selbst gefaltet und insbesondere in dieser Konfiguration lösbar fixiert wird.

12. Verfahren nach einem oder mehreren der Ansprüche 1 bis 10, wobei die einnutzigen Windelseitenteilbahnen (13a, 13b) um jeweils mindestens eine, insbesondere um jeweils mindestens zwei, weiter insbesondere um jeweils mindestens drei zu einer Längsrichtung (24) der jeweiligen einnutzigen Windelseitenteilbahn (13a, 13b) parallelen Faltlinien FW3 (76) oder FW4 (77) auf sich selbst gefaltet und insbesondere in dieser Konfiguration lösbar fixiert werden.

13. Verfahren nach einem oder mehreren der vorstehenden Ansprüche, **dadurch gekennzeichnet, dass** die Windelseitenteilabschnitte (14a, 14b) auf die Windelhauptteilbahn (15) gefaltet werden.

14. Verfahren nach einem oder mehreren der vorstehenden Ansprüche, **dadurch gekennzeichnet, dass** die vereinzelten Inkontinenzwegwerfwindeln (17) um mindestens eine, insbesondere zwei in einer Windelquerrichtung (74) erstreckte Faltlinien FQ gefaltet werden.

15. Verfahren nach einem oder mehreren der vorstehenden Ansprüche, wobei ein Quotient Q1 aus der Breite B1 und der Summe der Breiten B2 und B3 berechnet als: B1/(B2+B3) einen Wert von 0,2 bis 1,5, insbesondere einen Wert von 0,25 bis 1,2, insbesondere einen Wert von 0,25 bis 1,0, insbesondere einen Wert von 0,3 bis 0,9, insbesondere einen Wert von 0,4 bis 0,8 aufweist und/oder wobei ein Quotient Q2 aus der Breite B1 und der Summe der Breiten B4 und B5 berechnet als: B1/(B4+B5) einen Wert von 0,15 bis 1,5, insbesondere einen Wert von 0,2 bis 1,2, insbesondere einen Wert von 0,2 bis 1,0, insbesondere einen Wert von 0,25 bis 0,7, insbesondere einen Wert von 0,3 bis 0,5 aufweist.

16. Verfahren nach einem oder mehreren der vorstehenden Ansprüche, wobei gilt: B1≤(B2+B3) und/oder wobei gilt: B1≤(B4+B5).

## Claims

1. Method for producing disposable incontinence diapers (17), wherein a respective disposable incontinence diaper (17) has a diaper longitudinal direction (73) and a diaper transverse direction (74), and wherein the disposable incontinence diaper (17) has a main part (65) provided with an absorbent body (69), wherein the main part (65) has a back region (64) with a first rear lateral longitudinal edge (66) and with a second rear lateral longitudinal edge (67), and wherein there is joined to the first rear lateral longitudinal edge (66) a first elastic diaper side part (62), which extends in the diaper transverse direction (74) beyond the first rear lateral longitudinal edge (66) of the main part (65), and wherein there is joined to the second rear lateral longitudinal edge (67) a second elastic diaper side part (63), which extends in the diaper transverse direction (74) beyond the second rear lateral longitudinal edge (67) of the main part (65), said method comprising the following steps

   - conveying a first material web (3), a second material web (7), a third material web (8), a fourth material web (4) and a fifth material web (5), wherein the first material web (3) has a width B1 and a first material web edge region (30) and a second material web edge region (31) and comprises or consists of an attachment material that is substantially inextensible in a transverse direction (25) of the first material web (3), wherein the second material web (7) has a width B2 and a third material web edge region (32) and a fourth material web edge region (33) and comprises or consists of a first material that is elastic at least in part in a transverse direction (25) of the second material web (7), wherein the third material web (8) has a width B3 and a fifth material web edge region (34) and a sixth material web edge region (35) and comprises or consists of a second material that is elastic at least in part in a transverse direction (25) of the third material web (8), wherein the fourth material web (4) has a width B4 and a seventh material web edge region (38) and an eighth material web edge region (39) and comprises or consists of a first closure carrier material that is substantially inextensible in a transverse direction (25) of the fourth material web (4), and wherein the fifth material web (5) has a width B5 and a ninth material web edge region (40) and a tenth material web edge region (41) and comprises or consists of a second closure carrier material that is substantially inextensible in a transverse direction (25) of the fifth material web (5), and wherein the material web edge regions of the first (3), second (7), third (8), fourth (4) and fifth material web (5) are joined to each other in such a way that the first (3), second (7), third (8), fourth (4) and fifth material web (5) form a diaper side part web (10) of double use in its respective transverse direction (25), in such a way that a central region (53) of the double-use diaper side part web (10) is formed by the first material web (3), a first outer region of the double-use diaper side part web (10) is formed by the fourth material web (4), a second outer region of the double-use diaper side part web (10) is formed by the fifth material web (5), and a first elastic region arranged in the transverse direction (25) of the double-use diaper side part web (10) between the first outer region and the central region (53) is formed by the second material web (7), and a second elastic region arranged in the transverse direction (25) of the double-use diaper side part web (10) between the second outer region and the central region (53) is formed by the third material web (8),
- separating the double-use diaper side part web (10) in a longitudinal direction (24) of the double-use diaper side part web (10) and through the central region (53) of the double-use diaper side part web (10) in order to form a first diaper side part web (13a) and a second diaper side part web (13b), each of single use in their respective transverse direction (25),
- singulating diaper side part portions (14a, 14b) by separating the first (13a) and the second (13b) single-use diaper side part web in the respective transverse direction (25) of the first (13a) and the second (13b) single-use diaper side part web,
- feeding a diaper main part web (15) having a first diaper main part web edge and a second diaper main part web edge,
- joining the diaper side part portions (14a, 14b) to first regions (60) of the first diaper main part web edge and to second regions (61) of the second diaper main part web edge,
- singulating the disposable incontinence diapers (17) by separating the diaper main part web (15) in a transverse direction (25) of the diaper main part web (15).

2. Method according to Claim 1, wherein

- the third material web edge region (32) of the second material web (7) is joined to the first material web edge region (30) of the first material web (3), and wherein the fifth material web edge region (34) of the third material web (8) is joined to the second material web edge region (31) of the first material web (3), in such a way that the first (3), second (7) and third material web (8) together form a composite web (9), and wherein the composite web (9) has a first composite web edge region (36) and a second composite web edge region (37), and wherein
- the seventh material web edge region (38) (of the fourth material web (4)) is joined to the first composite web edge region (36) of the composite web (9), and wherein the ninth material web edge region (40) of the fifth material web (5) is joined to the second composite web edge region (37) of the composite web (9), in such a way that the composite web (9) together with the fourth (4) and fifth material web (5) form the diaper side part web (10) of double use in its transverse direction (25).

3. Method according to Claim 1 or 2, wherein the first (62) and the second elastic diaper side part (63) each have at least one closure element (44).

4. Method according to one or more of the preceding claims, wherein the second (7) and third material web (8) are made available by separating a first material web (6) of double use in its transverse direction (25).

5. Method according to one or more of the preceding claims, wherein the fourth (4) and fifth material web (5) are made available by separating a second material web of double use in its transverse direction.

6. Method according to one or more of Claims 1 to 4, wherein the first (3), fourth (4) and fifth material web (5) are made available by separating a material web (2) of triple use in its transverse direction (25).

7. Method according to one or more of the preceding claims, wherein the diaper side part portions (14a, 14b) are designed for single use in their longitudinal direction (24).

8. Method according to one or more of Claims 1 to 6, wherein the diaper side part portions are designed for double use in their longitudinal direction, and wherein the separation of the diaper main part web (15) is guided through the diaper side part portions in such a way that a first subportion of a respective diaper side part portion forms a rear diaper side part of a first disposable incontinence diaper (17), and a second subportion of the respective diaper side part portion forms a rear diaper side part of a second disposable incontinence diaper (17) immediately adjacent in a diaper longitudinal direction (73).

9. Method according to one or more of preceding Claims 3 to 8, wherein the respective closure elements (44) are joined to first regions of a first diaper side part web edge (42) and to second regions of a second diaper side part web edge (43).

10. Method according to one or more of the preceding claims, **characterized in that** the first (62) and the second elastic diaper side part (63) are folded (onto themselves) at in each case at least one (68), in particular at in each case at least two (68a, 68b), more particularly at in each case at least three (68a, 68b, 68c) fold lines FW1 (68, 68a, 68b, 68c), preferably extending parallel to the diaper longitudinal direction (73) of the disposable incontinence diaper (17), and in particular are fixed releasably in this configuration.

11. Method according to one or more of the preceding claims, wherein the double-use diaper side part web (10) is folded onto itself on both sides about in each case at least one, in particular about in each case at least two, more particularly about in each case at least three fold lines FW2 (50), parallel to the longitudinal direction (24) of the double-use diaper side part web (10), and in particular is fixed releasably in this configuration.

12. Method according to one or more of Claims 1 to 10, wherein the single-use diaper side part webs (13a, 13b) are folded onto themselves about in each case at least one, in particular about in each case at least two, more particularly about in each case at least three fold lines FW3 (76) or FW4 (77), parallel to a longitudinal direction (24) of the respective single-use diaper side part web (13a, 13b), and in particular are fixed releasably in this configuration.

13. Method according to one or more of the preceding claims, **characterized in that** the diaper side part portions (14a, 14b) are folded onto the diaper main part web (15).

14. Method according to one or more of the preceding claims, **characterized in that** the singulated disposable incontinence diapers (17) are folded about at least one, in particular two fold lines FQ extending in a diaper transverse direction (74).

15. Method according to one or more of the preceding claims, wherein a quotient Q1 from the width B1 and the sum of the widths B2 and B3 calculated as: B1/(B2+B3) has a value of 0.2 to 1.5, in particular a value of 0.25 to 1.2, in particular a value of 0.25 to 1.0, in particular a value of 0.3 to 0.9, in particular a value of 0.4 to 0.8, and/or wherein a quotient Q2 from the width B1 and the sum of the widths B4 and B5 calculated as: B1/(B4+B5) has a value of 0.15 to 1.5, in particular a value of 0.2 to 1.2, in particular a value of 0.2 to 1.0, in particular a value of 0.25 to 0.7, in particular a value of 0.3 to 0.5.

16. Method according to one or more of the preceding claims, wherein: $B1 \leq (B2+B3)$ and/or wherein: $B1 \leq (B4+B5)$.

**Revendications**

1. Procédé de fabrication de couches jetables pour incontinence (17), une couche jetable pour incontinence (17) respective présentant une direction longitudinale de couche (73) et une direction transversale de couche (74), et la couche jetable pour incontinence (17) présentant une partie principale (65) pourvue d'un corps absorbant (69), la partie principale (65) présentant une zone de dos (64) avec un premier bord longitudinal latéral arrière (66) et avec un deuxième bord longitudinal latéral arrière (67), et une première partie latérale de couche élastique (62) s'étendant dans la direction transversale de couche (74) au-delà du premier bord longitudinal latéral arrière (66) de la partie principale (65) étant jointe au premier bord longitudinal latéral arrière (66), et une deuxième partie latérale de couche élastique (63) s'étendant dans la direction transversale de la couche (74) au-delà du deuxième bord longitudinal latéral arrière (67) de la partie principale (65) étant jointe au deuxième bord longitudinal latéral arrière (67), comprenant les étapes suivantes :

- le transport d'une première bande de matériau (3), **d'une** deuxième bande de matériau (7), **d'une** troisième bande de matériau (8), d'une quatrième bande de matériau (4) et d'une cinquième bande de matériau (5), la première bande de matériau (3) présentant une largeur B1 et une première zone de bord de bande de matériau (30) et une deuxième zone de bord de bande de matériau (31) et comprenant un matériau de jonction essentiellement inextensible dans une direction transversale (25) de la première bande de matériau (3) ou étant constituée de celui-ci, la deuxième bande de matériau (7) présentant une largeur B2 et une troisième zone de bord de bande de matériau (32) et une quatrième zone de bord de bande de matériau (33) et comprenant un premier matériau élastique au moins par zones dans une direction transversale (25) de la deuxième bande de matériau (7) ou étant constituée de celui-ci, la troisième bande de matériau (8) présentant une largeur B3 et une cinquième zone de bord de bande de matériau (34) et une sixième zone de bord de bande de matériau (35) et comprenant un deuxième matériau élastique au moins par zones dans une direction transversale (25) de la troisième bande de matériau (8) ou étant constituée de celui-ci, la quatrième bande de matériau (4) présentant une largeur B4 et une septième zone de bord de bande de matériau (38) et une huitième zone de bord de bande de matériau (39) et comprenant un premier matériau de support de fermeture essentiellement inextensible dans une direction transversale (25) de la quatrième bande de matériau (4) ou étant constituée de celui-ci, et la cinquième bande de matériau (5) présentant une largeur B5 et une neuvième zone de bord de bande de matériau (40) et une dixième zone de bord de bande de matériau (41) et comprenant un deuxième matériau de support de fermeture essentiellement inextensible dans une direction transversale (25) de la cinquième bande de matériau (5) ou étant constituée de celui-ci, et les zones de bord de bande de matériau des première (3), deuxième (7), troisième (8), quatrième (4) et cinquième bandes de matériau (5) étant jointes les unes aux autres de telle sorte que les première (3), deuxième (7), troisième (8), quatrième (4) et cinquième bandes de matériau (5) forment une bande de partie latérale de couche à deux usages (10) dans leur direction transversale respective (25), de telle sorte qu'une zone centrale (53) de la bande de partie latérale de couche à deux usages (10) est formée par la première bande de matériau (3), qu'une première zone extérieure de la bande de partie latérale de couche à deux usages (10) est formée par la quatrième bande de matériau (4), qu'une deuxième zone extérieure de la bande de partie latérale de couche à deux usages (10) est formée par la cinquième bande de matériau (5), et qu'une première zone élastique agencée dans la direction transversale (25) de la bande de partie latérale de couche à deux usages (10) entre la première zone extérieure et la zone centrale (53) est formée par la deuxième bande de matériau (7), et qu'une deuxième zone élastique agencée dans la direction transversale (25) de la bande de partie latérale de couche à deux usages (10) entre la deuxième zone extérieure et la zone centrale (53) est formée par la troisième bande de matériau (8),

- la séparation de la bande de partie latérale de couche à deux usages (10) dans une direction longitudinale (24) de la bande de partie latérale de couche à deux usages (10) et à travers la zone centrale (53) de la bande de partie latérale de couche à deux usages (10), pour former une première (13a) et une deuxième bande de partie latérale de couche à un usage (13b) respectivement dans leur direction transversale respective (25),
- l'individualisation de sections de partie latérale de couche (14a, 14b) par séparation de la première (13a) et de la deuxième (13b) bande de partie latérale de couche à un usage dans la direction transversale respective (25) de la première (13a) et de la deuxième (13b) bande de partie latérale de couche à un usage,
- l'amenée d'une bande de partie principale de couche (15) ayant un premier bord de bande de partie principale de couche et un deuxième bord de bande de partie principale de couche,
- la jonction des sections de partie latérale de couche (14a, 14b) à des premières zones (60) du premier bord de bande de partie principale de couche et à des deuxièmes zones (61) du deuxième bord de bande de partie principale de couche,
- l'individualisation des couches jetables pour incontinence (17) par séparation de la bande de partie principale de couche (15) dans une direction transversale (25) de la bande de partie principale de couche (15).

2. Procédé selon la revendication 1,

- la troisième zone de bord de bande de matériau (32) de la deuxième bande de matériau (7) étant jointe à la première zone de bord de bande de matériau (30) de la première bande de matériau (3), et la cinquième zone de bord de bande de matériau (34) de la troisième bande de matériau (8) étant jointe à la deuxième zone de bord de bande de matériau (31) de la première bande de matériau (3), de telle sorte que la première (3), la deuxième (7) et la troisième (8) bande de matériau forment conjointement une bande composite (9), et la bande composite (9) présentant une première zone de bord de bande composite (36) et une deuxième zone de bord de bande composite (37), et
- la septième zone de bord de bande de matériau (38) (de la quatrième bande de matériau (4)) étant jointe à la première zone de bord de bande composite (36) de la bande composite (9), et la neuvième zone de bord de bande de matériau (40) de la cinquième bande de matériau (5) étant jointe à la deuxième zone de bord de bande composite (37) de la bande composite (9), de telle sorte que la bande composite (9) forme, conjointement avec la quatrième (4) et la cinquième bande de matériau (5), la bande de partie latérale de couche à deux usages (10) dans sa direction transversale (25).

3. Procédé selon la revendication 1 ou 2, la première (62) et la deuxième (63) partie latérale de couche élastique présentant chacune au moins un élément de fermeture (44).

4. Procédé selon une ou plusieurs des revendications précédentes, la deuxième (7) et la troisième (8) bande de matériau étant fournies par séparation d'une première bande de matériau à deux usages (6) dans sa direction transversale (25).

5. Procédé selon une ou plusieurs des revendications précédentes, la quatrième (4) et la cinquième (5) bande de matériau étant obtenues par séparation d'une deuxième bande à deux usages dans sa direction transversale.

6. Procédé selon une ou plusieurs des revendications 1 à 4 précédentes, la première (3), la quatrième (4) et la cinquième (5) bande de matériau étant fournies par séparation **d'une** bande de matériau à trois usages (2) dans sa direction transversale (25).

7. Procédé selon une ou plusieurs des revendications précédentes, les sections de partie latérale de couche (14a, 14b) étant configurée à un usage dans leur direction longitudinale (24).

8. Procédé selon une ou plusieurs des revendications 1 à 6, les sections de partie latérale de couche étant configurées à deux usages dans leur direction longitudinale, et la séparation de la bande de partie principale de couche (15) étant guidée à travers les sections de partie latérale de couche de telle sorte qu'une première section partielle d'une section de partie latérale de couche respective forme une partie latérale de couche arrière d'une première couche jetable pour incontinence (17) et qu'une deuxième section partielle de la section de partie latérale de couche respective forme une partie latérale de couche arrière d'une deuxième couche jetable pour incontinence (17) directement adjacente dans une direction longitudinale de couche (73).

9. Procédé selon une ou plusieurs des revendications 3 à 8 précédentes, les éléments de fermeture respectifs (44) étant joints à des premières zones d'un premier bord de bande de partie latérale de couche (42) et à des deuxièmes zones d'un deuxième bord de bande de partie latérale de couche (43).

10. Procédé selon une ou plusieurs des revendications précédentes, **caractérisé en ce que** la première (62) et la deuxième (63) partie latérale de couche élastique sont pliées (sur elles-mêmes) respectivement au niveau d'au moins une (68), en particulier respectivement au niveau d'au moins deux (68a, 68b), plus particulièrement respectivement au niveau d'au moins trois (68a, 68b, 68c) lignes de pliage FW1 (68, 68a, 68b, 68c) s'étendant de préférence parallèlement à la direction longitudinale de couche (73) de la couche jetable pour incontinence (17), et sont en particulier fixées de manière amovible dans cette configuration.

11. Procédé selon une ou plusieurs des revendications précédentes, la bande de partie latérale de couche à deux usages (10) étant pliée sur elle-même des deux côtés respectivement autour d'au moins une, en particulier respectivement autour d'au moins deux, plus particulièrement respectivement autour d'au moins trois lignes de pliage FW2 (50) parallèles à la direction longitudinale (24) de la bande de partie latérale de couche à deux usages (10), et étant en particulier fixée de manière amovible dans cette configuration.

12. Procédé selon une ou plusieurs des revendications 1 à 10, les bandes de partie latérale de couche à un usage (13a, 13b) étant pliées sur elles-mêmes respectivement autour d'au moins une, en particulier respectivement autour d'au moins deux, et plus particulièrement respectivement autour d'au moins trois lignes de pliage FW3 (76) ou FW4 (77) parallèles à une direction longitudinale (24) de la bande de partie latérale de couche à un usage (13a, 13b) respective, et étant en particulier fixées de manière amovible dans cette configuration.

13. Procédé selon une ou plusieurs des revendications précédentes, **caractérisé en ce que** les sections de partie latérale de couche (14a, 14b) sont pliées sur la bande de partie principale de couche (15).

14. Procédé selon une ou plusieurs des revendications précédentes, **caractérisé en ce que** les couches jetables pour incontinence (17) individualisées sont pliées autour d'au moins une, en particulier deux lignes de pliage FQ s'étendant dans une direction transversale de couche (74).

15. Procédé selon une ou plusieurs des revendications précédentes, un quotient Q1 de la largeur B1 et de la somme des largeurs B2 et B3 calculé comme : B1/(B2+B3) présentant une valeur de 0,2 à 1,5, en particulier une valeur de 0,25 à 1,2, en particulier une valeur de 0,25 à 1,0, en particulier une valeur de 0,3 à 0,9, en particulier une valeur de 0,4 à 0,8, et/ou un quotient Q2 de la largeur B1 et de la somme des largeurs B4 et B5 calculé comme : B1/(B4+B5) présentant une valeur de 0,15 à 1,5, en particulier une valeur de 0,2 à 1,2, en particulier une valeur de 0,2 à 1,0, en particulier une valeur de 0,25 à 0,7, en particulier une valeur de 0,3 à 0,5.

16. Procédé selon une ou plusieurs des revendications précédentes, avec : B1 ≤ (B2+B3) et/ou avec : B1 ≤ (B4+B5).

Fig. 1

EP 3 840 706 B1

Fig. 2a

Fig. 2b

Fig. 3a

Fig. 3 b

Fig. 3c

Fig. 4

Fig. 5

Fig. 6

Fig. 7

Fig. 8

Fig. 9

Fig. 10a

Fig. 10b

Fig. 10c

Fig. 11

Fig.12a

Fig.12b

**EP 3 840 706 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2008036706 A1
- WO 2011101773 A1
- WO 9603952 A1
- WO 9631180 A1
- WO 2007042084 A1
- EP 0650714 A1
- WO 2017140604 A1